# EUROPÄISCHE PATENTSCHRIFT

(11) **EP 2 641 903 B1**
(45) Veröffentlichungstag und Bekanntmachung des Hinweises auf die Patenterteilung: **22.10.2014**
(21) Anmeldenummer: 12160062.1
(22) Anmeldetag: 19.03.2012
(51) Int. Cl.: C07D 307/80

(54) **Dihydrobenzofuran-Derivate als Riech- und/oder Aromastoffe**
Dihydrobenzofuran derivatives as olfactory and or aroma substances
Dérivés de dihydrobenzofuranes en tant que parfums et/ou arômes

(43) Veröffentlichungstag der Anmeldung: 25.09.2013
(73) Patentinhaber: Symrise AG, 37603 Holzminden (DE)
(72) Erfinder: Oertling, Heiko, 37603 Holzminden (DE); Gömann, Claudia, 37640 Golmbach-Warbsen (DE); vom Ende, Marc, 13585 Berlin (DE)
(74) Vertreter: Fabry, Bernd

(56) Entgegenhaltungen:
- WO-A1-2007/030061
- KRAFT PHILIP ED - DAVID J ROWE: "Aroma Chemicals IV: Musks", 3. März 2009 (2009-03-03), CHEMISTRY AND TECHNOLOGY OF FLAVORS AND FRAGRANCES, BLACKWELL PUBLISHING LTD, PAGE(S) 143 - 168, XP002671050, ISBN: 978-1-4443-0551-7 * Abbildung 7.3 *
- SHI, G., COTTENS,S., SHIBA, S., SCHLOSSER,M.: "The Diels-Alder Approach to Musk Odor type Arenes", TETRAHEDRON, Bd. 48, Nr. 48, 1992, Seiten 10569-10574, XP002677231,
- DADUSH ET AL: "Microwave activation of aluminia and its use as a catalyst in synthetic reactions", JOURNAL OF CHEMICAL RESEARCH, SCIENCE REVIEWS 2000 LTD, 1. Januar 2009 (2009-01-01), Seiten 120-123, XP009159883,

## Beschreibung

Die vorliegende Erfindung betrifft die Verwendung einzelner Verbindungen der Formel (I) 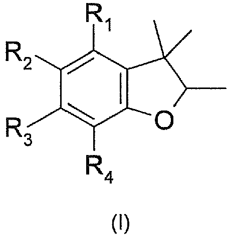 oder Mischungen davon, insbesondere Mischungen aus zwei, drei, vier, fünf, sechs oder mehreren verschiedenen Verbindungen der Formel (I) als Riech- und/oder Aromastoff bzw. als Riechstoff- und/oder Aromastoffmischung. Die Reste R₁ bis R₄ haben die im Rahmen des vorliegenden Textes beschriebene Bedeutung.

Die Erfindung betrifft auch neue Riechstoff- und/oder Aromastoffmischungen, die neben einer Verbindung der Formel (I) oder einer Mischung davon, wie jeweils hierin beschrieben, einen oder mehrere, insbesondere zwei, drei oder mehrere, weitere Riech- und/oder Aromastoffe, die keine Verbindungen der Formel (I) sind, umfassen oder daraus bestehen.

Weiterhin betrifft die vorliegende Erfindung parfümierte und/oder aromatisierte Artikel, die eine Riechstoff- und/oder Aromastoffmischung gemäß der vorliegenden Erfindung enthalten.

Im Rahmen der vorliegenden Erfindung werden auch gegenüber dem Stand der Technik neue, besonders vorteilhafte Verbindungen der Formel (I) und Mischungen davon beschrieben.

Die vorliegende Erfindung betrifft auch ein Verfahren zum Vermitteln, Modifizieren und/oder Verstärken eines Geruchs und/oder Geschmacks, vorzugsweise eines moschusartigen und/oder holzigen Geruchs und/oder Geschmacks.

Weitere Aspekte der vorliegenden Erfindung ergeben sich aus der nachfolgenden Beschreibung sowie insbesondere den beigefügten Patentansprüchen.

Trotz einer Vielzahl bereits vorhandener Riech- und Aromastoffe besteht in der Parfümindustrie weiterhin ein genereller Bedarf an neuen Riech- und Aromastoffen. So besteht insbesondere ein Bedarf an Riechstoffen mit moschus Duftnoten, insbesondere solchen Riechstoffen, die in der Lage sind, neben einer moschus Duftnote (in Riechstoffmischungen) weitere interessante Geruchsnoten zu erzeugen und mit ihren neuartigen bzw. originellen Dufteigenschaften die Möglichkeiten des Parfümeurs zu erweitern. Insbesondere besteht ein Interesse an Riechstoffen mit moschus Duftnoten, welche in der Lage sind, eine harmonische Kombination mit holzig und/oder blumig duftenden Riechstoffen und/oder weiteren moschus Riechstoffen einzugehen. Vorzugsweise sollte eine Überlagerung der unterschiedlichen geruchlichen Aspekte und Noten erfolgen, um dadurch einen insgesamt komplexen Geruchseindruck zu erzeugen.

Für die Kreation neuartiger moderner Kompositionen besteht ständiger Bedarf an Riechstoffen mit besonderen (geruchlichen) Eigenschaften, die geeignet sind, als Grundlage für die Komposition von neuartigen modernen Parfüms mit komplexem Geruchscharakter zu dienen. Bevorzugte Riechstoffe sollten neben einer moschus Duftnote weitere Noten und/oder Aspekte bzw. Eigenschaften aufweisen, die ihnen einen besonderen geruchlichen Charakter und Komplexität sowie idealerweise weitere positive Eigenschaften verleihen.

Die dieser Erfindung zugrunde liegende Aufgabe bestand grundsätzlich darin, Riech- und/oder Aromastoffe mit moschus (Duft-)Noten bereitzustellen, die vorzugsweise weitere interessante und originelle (geruchliche) Eigenschaften aufweisen, so dass neuartige und originelle Riechstoff- und/oder Aromastoffmischungen mit besonderen (geruchlichen) Noten und Aspekten hergestellt werden können. Insbesondere galt es Riechstoffe mit moschus Duftnoten anzugeben, die vor allem zur Kombination mit weiteren Riechstoffen geeignet sind, z.B. zur Kombination mit weiteren Riechstoffen, die eine blumige Duftnote besitzen.

Bevorzugte Aufgabe der vorliegenden Erfindung war es, Aroma- und/oder Riechstoffe bereitzustellen, die neben ihren vorteilhaften primären, insbesondere geruchlichen, Eigenschaften zusätzlich vorteilhafte Sekundäreigenschaften besitzen, z.B. eine höhere Stabilität unter bestimmten Anwendungsbedingungen, eine höhere Ausgiebigkeit, ein besseres Haftungsvermögen, eine hohe Substantivität, eine vorteilhafte Booster-Wirkung und/oder ein starkes Blooming, so dass sensorisch vorteilhafte Effekte und/oder bessere dermatologische und toxikologische Eigenschaften gegenüber vergleichbaren Riech- bzw. Aromastoffen erzielt werden können.

Besonders bevorzugte Aufgabe der vorliegenden Erfindung war es, Aroma- bzw. Riechstoffe bereitzustellen, die neben ihren sensorischen Eigenschaften eine verbesserte Bioabbaubarkeit und reduzierte Bioakkumulation aufweisen. Denn insbesondere die Klasse der polycyclischen Moschusriechstoffe (PCMs), zu der kommerziell erfolgreiche Vertreter der synthetischen Moschusriechstoffe gehören, leidet an unzureichender Bioabbaubarkeit. Als Konsequenz dieser unzureichenden Degradibilität reichern sich solche Stoffe in der Umwelt an und werden unter anderem über die Nahrungskette in diversen Organismen angereichert bzw. akkumuliert (Bioakkumulation). Aufgrund der Lipophilie dieser Stoffe kann man sie im Fettgewebe von Säugetieren nachweisen (siehe z.B.: *"Polymoschus-Verbindungen in Humanblut II - Humanbiomonitoring von Moschusduft-stoffen",* M. Uhl (Umweltbundesamt GmbH Österreich), H. P. Hutter (Institut für Umwelthygiene, Public Health, Medizinische Universität Wien), G. Lorbeer (Analytik All, Umweltbundesamt GmbH Österreich)).

Ein Bespiel aus der Gruppe der polyzyklischen Moschusverbindungen ist Galaxolide^{®} (auch HHCB genannt, C₁₈H₂₆O, CAS-Nummer: 1222-05-5, Quelle: Umweltprobenbank des Bundes). HHCB ist die derzeit weltweit am häufigsten verwendete Substanz aus der Gruppe der polyzyklischen Moschusverbindungen. HHCB ist eine polyzyklische Moschusverbindung, die als synthetischer Duftstoff beispielsweise in Kosmetika und Körperpflegemitteln wie Seifen, Shampoos, Lotionen, Deodorants, in Wasch- und Reinigungsmitteln, Lufterfrischern, Papier und Textilien eingesetzt wird. Nach Gebrauch gelangt HHCB über Abwässer und Klärschlämme in die Umwelt, wo es nur langsam abgebaut wird. HHCB ist lipophil und reichert sich in Organismen an (Bioakkumulation).

Eine schlechte Bioabbaubarkeit und die Tendenz zur Bioakkumulation sind direkt mit den Stoffeigenschaften, wie beispielsweise der Wasserlöslichkeit, der Lipophilie und dem Verteilungskoeffizienten in Octanol/Wasser, verknüpft. Diese Eigenschaften wiederum resultieren aus der chemischen Struktur eines Stoffes.

Generell lässt sich festhalten, dass in den Kohlenstoffgerüsten der gängigen Riechstoffe eine Sauerstoffsubstitution (also Austausch eines Kohlenstoffatoms gegen ein Sauerstoffatom) eine bessere bzw. schnellere Bioabbaubarkeit begünstigt oder eine solche erst ermöglicht. Durch den Austausch erreicht man eine höhere Polarität des Stoffes und damit eine geringere Lipophilie. Es ist anzunehmen, dass höher sauerstoff-substituierte Stoffe auch weniger bioakkumulieren.

Zusammengefasst bestand die primäre Aufgabe der vorliegenden Erfindung in der Bereitstellung von vorzugsweise bioabbaubaren Riech- und/oder Aromastoffen mit moschus (Duft-)Noten und weiteren, vorteilhaften Eigenschaften, wie vorstehend beschrieben.

Eine weitere Aufgabe bestand in der Bereitstellung neuer Riech- und/oder Aromastoffe, neuer Riechstoff- und/oder Aromastoffmischungen sowie neuer parfümierter und/oder aromatisierter Produkte.

Weitere Aufgaben der vorliegenden Erfindung ergeben sich aus der nachfolgenden Beschreibung sowie insbesondere den beigefügten Patentansprüchen.

Die primäre Aufgabe der vorliegenden Erfindung wird gelöst durch die Verwendung
- einer Verbindung der Formel (I) 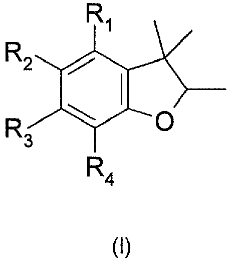 oder
- einer Mischung aus zwei, drei, vier, fünf, sechs oder mehreren verschiedenen Verbindungen der Formel (I),
wobei für die Reste der Verbindung der Formel (I) bzw. die Reste sämtlicher Verbindungen der Formel (I) jeweils gilt, dass R₁, R₂, R₃ und R₄ jeweils unabhängig voneinander Wasserstoff, Methyl, Ethyl, Propyl, Isopropyl, Butyl, sec-Butyl, tert-Butyl, iso-Butyl, Acetyl, Formyl oder Cyano bedeuten,
als Riech- und/oder Aromastoff bzw. als Riechstoff- und/oder Aromastoffmischung, insbesondere als Riechstoff bzw. als Riechstoffmischung.

Im Rahmen der vorliegenden Erfindung wurden auch gegenüber dem Stand der Technik neue Verbindungen der obigen Formel (I) sowie Mischungen davon gefunden. Solche sind weiter unten beschrieben.

Die erfindungsgemäß zu verwendenden Verbindungen sind vorteilhafterweise in der Lage, sowohl den parfümistischen Anforderungen zu genügen, als auch eine besonders vorteilhafte Bioabbaubarkeit zu besitzen.

Es war besonders überraschend, dass sich gerade die hierin beschriebenen Verbindungen der Formel (I) für die Zwecke der vorliegenden Erfindung eignen. Denn die Suche nach geeigneten Riechstoffen, wurde - und wird üblicherweise - durch folgende Gegebenheiten erschwert:
- Die Mechanismen der Geruchswahrnehmung sind (noch) nicht ausreichend bekannt.
- Die Zusammenhänge zwischen der speziellen Geruchswahrnehmung einerseits und der chemischen Struktur des zugehörigen Riechstoffs andererseits sind (noch) nicht hinreichend erforscht.
- Häufig bewirken bereits geringfügige Änderungen am strukturellen Aufbau eines bekannten Riechstoffs starke Änderungen der sensorischen (oder anderer) Eigenschaften. Diese können zudem die Verträglichkeit für den menschlichen Organismus beeinträchtigen.

Es ist daher in der Regel nicht oder nur begrenzt vorhersehbar, wie sich strukturelle Änderungen auf die sensorischen (sowie gegebenenfalls auf weitere) Eigenschaften einer Substanz auswirken.

Die Verbindungen der Formel (I), wie hierin beschrieben, sind nicht nur grundsätzlich höher sauerstoff-substituiert als bislang kommerziell verwendete Stoffe, sondern (auch bzw. insbesondere) am schwer zu oxidierenden aromatischen Kern sauerstoffsubstituiert. Der Abbau aromatischer Kohlenwasserstoffverbindungen in der freien Umwelt beginnt in der Regel mit einem oxidativen Schritt, der durch den aeroben Abbau durch Mikroorganismen induziert wird. Durch die Sauerstoffsubstitution am aromatischen Kern kann eine (weitergehende) mikrobielle Metabolisierung (zum Brenzkatechin) im Unterschied zu nicht oxidierten Stoffen vorteilhafterweise mittels einer Phenol-Monoxygenase anstelle einer Dioxygenase vonstatten gehen. Der aerobe Abbau von phenolischen Aromaten geht daher in der Regel leichter und schneller vonstatten als der aerobe Abbau von nicht oxidierten Stoffen (siehe hierzu z.B. *"*Mikrobiologische Umsetzung ausgewählter Stoffgruppen: aromatische Kohlenwasserstoffe" in "Chemie und Biologie der Altlasten", herausgegeben von der Fachgruppe Wasserchemie der GdCh, VCH, Weinheim, 1997).

Es war - insbesondere aufgrund der vorstehend genannten, erschwerenden Gegebenheiten bei der Suche nach geeigneten Riechstoffen - überraschend, dass die erfindungsgemäß zu verwendenden Verbindungen der Formel (I) neben einer vorteilhaften Bioabbaubarkeit auch besonders vorteilhafte sensorische Eigenschafte besitzen. Weiterhin besitzen diese Verbindungen über ihre primären, insbesondere geruchlichen, Eigenschaften hinaus zusätzlich über (weitere) positive Sekundäreigenschaften. Beispielsiwese besitzen oder vermitteln sie ein besseres Haftungsvermögen und eine hohe Substantivität (gegenüber Riechstoffen mit ähnlichen Geruchseigenschaften). Die erfindungsgemäß zu verwendenden Verbindungen bzw. die entsprechenden Mischungen besitzen vorteilhafterweise auch eine hohe Stabilität unter verschiedenen Anwendungsbedingungen und weisen eine hohe Ausgiebigkeit auf.

Die Verbindungen der Formel (I) können gegebenenfalls jeweils als reine Stereoisomere oder als Mischung von Stereoisomeren vorliegen. Insbesondere kann eine Verbindung der Formel (I) als Enantiomer oder Mischung von Enantiomeren vorliegen.

Die erfindungsgemäß zu verwendenden Verbindungen der Formel (I) weisen besonders vorteilhafte moschusartige Noten auf. Dementsprechend ist im Rahmen der vorliegenden Erfindung eine Verwendung einer hierin beschriebenen Verbindung der Formel (I) bzw. einer Mischung mehrerer unterschiedlicher Verbindungen der Formel (I) besonders bevorzugt, wobei die Verbindung bzw. die Mischung als Riech- und/oder Aromastoff mit moschusartiger Note bzw. als Riechstoff- und/oder Aromastoffmischung mit moschusartiger Note verwendet wird.

Besonders bevorzugt ist eine erfindungsgemäße Verwendung einer hierin beschriebenen Verbindung der Formel (I) bzw. einer Mischung mehrerer unterschiedlicher Verbindungen der Formel (I) zum Vermitteln, Modifizieren und/oder Verstärken eines Geruchs und/oder eines Geschmacks, vorzugsweise eines moschusartigen Geruchs, insbesondere eines strahlend-moschusartigen Geruchs, eines exaltierenden Geruchs und/oder eines holzigen, insbesondere eines weich holzigen Geruchs.

Grundsätzlich können die sensorischen Eigenschaften, insbesondere die Geruchseigenschaften, erfindungsgemäß zu verwendender Verbindungen der Formel (I) vor allem wie folgt beschrieben werden: stark strahlend, moschus-artig, exaltierend, weich, an Moschuskörneröl erinnernd, sehr natürlicher (Geruchs-)Eindruck. Die erfindungsgemäß zu verwendenden Verbindungen der Formel (I) bzw. entsprechende Mischungen können vorteilhafterweise insbesondere zum Vermitteln, Modifizieren und/oder Verstärken einer entsprechenden Geruchs- und/oder Geschmacksnote eingesetzt werden.

In Anbetracht des Standes der Technik gilt das hierin beschriebene Gebiet der Riechstoffchemie als gut untersucht (z.B. im Zusammenhang mit den Produkten Traseolide®, Celestolide® und Phantolide®). Daher war es besonders überraschend, dass nunmehr neue, besonders wertvolle Riechstoffe gefunden bzw. als erfindungsgemäß geeignet identifiziert werden konnten. Darüber hinaus verfügen die erfindungsgemäß zu verwendenden Verbindungen der Formel (I), insbesondere die hierin beschriebenen gegenüber dem Stand der Technik neuen Verbindungen der Formel (I), gegenüber bisherigen Riechstoffen über eigenständige olfaktorische Eigenschaften, die sich deutlich von den bekannten Riechstoffen abheben und diese zum Teil sogar übertreffen.

Die Geruchsbeschreibungen von Traseolide®, Celestolide® und Phantolide® lassen sich wie folgt zusammenfassen:
Celestolide®: starke Moschusnote
Traseolide®: holzig, moschusartig
Phantolide®: moschusartig

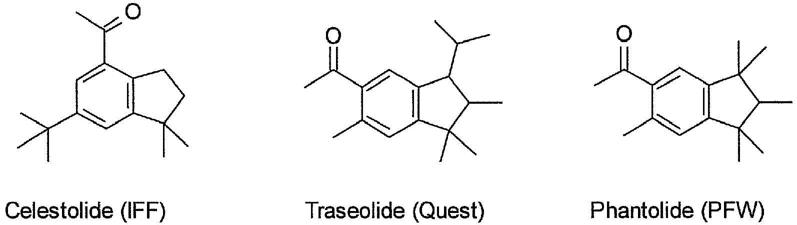

Für die erfindungsgemäß zu verwendenden Verbindungen der Formel (I) ist das Methylierungsmuster am Fünfring (siehe oben) charakteristisch. Weiterhin ist ein Kohlenstoffatom des Fünfrings, wie vorstehend beschrieben, (sauerstoff-)substituiert.

Strukturell ähnliche Verbindungen sind in WO 96/03396 beschrieben. Im Unterschied zu den erfindungsgemäß zu verwendenden Verbindungen der Formel (I) fehlt den darin beschriebenen Verbindungen jedoch eine Methylgruppe in der 2-Position, wie an folgendem Beispiel zu sehen ist:

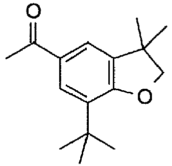

In WO 96/03396 werden außerdem keine sensorischen Eigenschaften dieser Verbindungen beschrieben. Vielmehr wird auf die anti-inflammatorischen Eigenschaften der darin beschriebenen Substanzen abgehoben.

Dies gilt auch für folgende Verbindung, die in WO 97/28148 als Zwischenstufe in einer Synthesesequenz erwähnt ist:

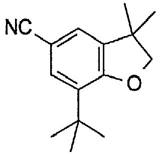

In WO2007/030061 sind folgende Verbindungen als Syntheseintermediate in einer 1 : 1 Mischung beschrieben:

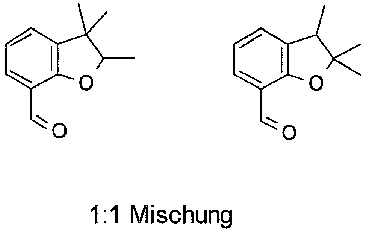

Die sensorischen Eigenschaften der erfindungsgemäß zu verwendenden Verbindungen sind im Stand der Technik nicht erkannt oder nahegelegt worden.

In US 3551456 ist die Eignung von Coumaran- und Chroman-Derivaten zur Verwendung als Riech- und/oder Aromastoffe beschrieben. Beschrieben wird beispielsweise die Kondensation von Phenol, monoalkylierten Phenolen und symmetrisch substituierten Bisalkylphenolen (z.B. 2,4-Dipropylphenol) unter sauren Bedingungen (z.B. mit Phosphorsäure, Schwefelsäure oder einer Lewis-Säure) mit einem konjugierten Dien (z.B. Butadien, Pentadien oder Hexadien).

Die Kondensation erfolgt naturgemäß nicht regioselektiv, und es werden Gemische der polyalkylierten Coumarane und Chromane erhalten. Diese werden gemäß US 3551456 nicht destillativ aufgetrennt. Anschließend werden die erhaltenen Gemische unter Friedel-Crafts Bedingungen gemäß US 3551456 acetyliert.

Die erhaltenen Mischungen werden olfaktorisch mit einem Moschus-Charakter beschrieben. Als Einzelsubstanzen mit einem acetylierten Coumarangerüst sind in US 3551456 folgende Isomere aufgeführt:

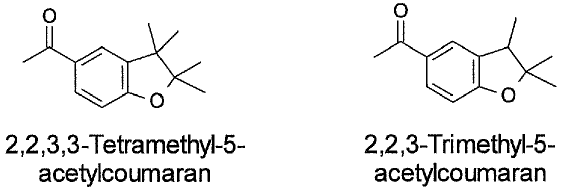

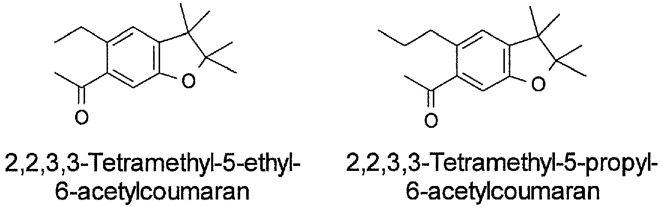

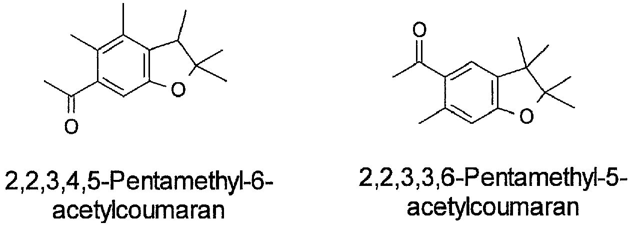

Keine der in US 3551456 konkret beschriebenen Verbindungen weist jedoch das für die erfindungsgemäß zu verwendenden Verbindungen der Formel (I) charakteristische 2,3,3-Trimethyl-(coumaran-)Substitutionsmuster auf.

Unter den in US 3551456 beschriebenen Reaktionsbedingungen können darüber hinaus generell keine Verbindungen der Formel (I) erhalten werden.

Gemäß "Example II" von US 3551456 werden zwar durch direkte, saure Kondensation von Phenol mit Isopren durch Phosphorsäure Gemische von Trimethylcoumaranen, die nicht näher spezifiziert werden, erhalten. Nach Acetylierung unter Friedel-Crafts-Bedingungen und anschließender Destillation werden allerdings 2,2,3-Trimethylacetylcoumarane erhalten, die nicht der Formel (I) entsprechen.

Die saure Kondensation von Phenol mit Isopren und Phosphorsäure ist eine in der chemischen Literatur gründlich untersuchte Reaktion. Exemplarisch verweisen wir diesbezüglich auf nachfolgende Literaturstellen:
- L. Claisen, Chemische Berichte 1921, 54, 200 ff:
   beschrieben werden 2,2-Dimethylchromanderivate
- Bader et al., Journal of the American Chemical Society 1958, 80, 3073 ff:
   beschrieben werden die Umsetzung mit 71%iger Phosphorsäure in Toluol bei 20°C für 16 Stunden, Chroman-Derivate sowie ortho- und para-Phenolderivate
- GB 906483, Bayer, 1962:
   beschrieben wird die Umsetzung von Phenol mit Isopren unter Aluminium-Phenoxid-Katalyse, wobei folgende Isomere erhalten werden:

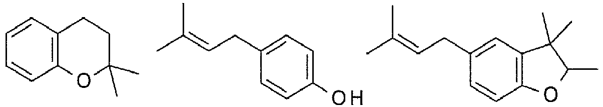

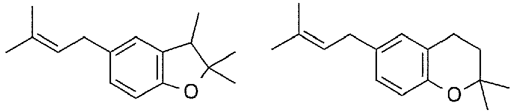

Weiterhin wird die Umsetzung von para-Kresol mit Isopren unter Aluminium-Phenoxid-Katalyse beschrieben, wobei folgende Isomere erhalten werden: 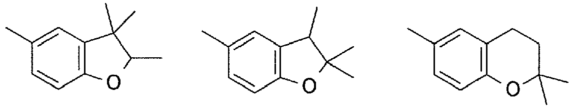
- K. C. Dewhirst, F. F. Rust, Journal of Organic Chemistry 1963, 28, 798 ff.:
   beschrieben wird die Umsetzung von Phenol mit Isopren unter Aluminium-Phenoxid-Katalyse, wobei keine Coumaran-Derivate gebildet werden
- E. A. Vdovtsova, Zhournal Organicheskoi Khimii 1965, vol 1, 2192 ff.:
   beschrieben wird die Umsetzung mit 71 %iger und 100%iger Phosphorsäure ohne Lösungsmittel; es werden keine Coumaran-Derivate beschrieben
- In US 4107324, Bayer, 1978 wird erwähnt, dass man Indan-Derivate durch Kondensation von Phenol und Isopren mit Phosphorsäure erhalten kann.
- D. M. X. Donnelly et al., Journal of Chemical Research, Synopses (1980), (1), 1, 127 ff.:
   beschrieben wird eine Umsetzung mit 71%iger Phosphorsäure, wobei ein orthoprenyliertes Derivat erhalten wird
- J. P. Ward et al., Chemistry & Industry 1989, 345 ff.:
   beschrieben wird die Umsetzung von Phenol und Isopren mit Aluminiumphenolat, wobei Chroman und para-Phenolderivat erhalten werden
- WO 2007/000582:
   beschrieben werden Phenol, Phosphorpentoxid und Phosphorsäure in meta-Xylol, woraus sich Indenol ergibt
- King Kuok (Mimi) Hii et al. Chem. Commun. 2008, 2325 ff.:
   beschrieben wird eine Umsetzung mit Kupfertriflat zu Chromanderivat
- Hintermann et al. Journal of Organic Chemistry 2011, 76, 9353 ff.:
   beschrieben wird die Umsetzung von Phenol mit Isopren und verschiedenen Lewis-Säuren (Triflat-Salzen) zu Chromanen

Es war besonders überraschend, dass die Verbindungen der Formel (I) sehr nützliche sensorische (sowie weitere positive) Eigenschaften besitzen, wie hierin beschrieben, und in Parfümformulierungen grundsätzlich eine starke Wirkung zeigen.

Im Folgenden werden erfindungsgemäß besonders bevorzugte und für die Zwecke der vorliegenden Erfindung besonders geeignete Verbindungen und Mischungen beschrieben.

Erfindungsgemäß bevorzugt ist eine Verwendung (wie oben beschrieben), wobei für die Verbindung der Formel (I) bzw. eine, mehrere oder sämtliche Verbindungen der Formel (I) jeweils unabhängig voneinander gilt:
R₁ bedeutet Wasserstoff, Methyl, Acetyl oder Formyl, vorzugsweise Wasserstoff oder Methyl,
   und/oder
R₂ bedeutet Methyl, Formyl oder Acetyl, vorzugsweise Formyl oder Acetyl,
   und/oder
R₃ bedeutet Wasserstoff oder Methyl
   und/oder
R₄ bedeutet Methyl, Ethyl, Propyl, Isopropyl, Butyl, sec-Butyl, tert-Butyl oder iso-Butyl, vorzugsweise Methyl, Ethyl, Isopropyl oder tert-Butyl.

Besonders bevorzugt ist eine Verwendung, wobei für die Verbindung der Formel (I) bzw. eine, mehrere oder sämtliche Verbindungen der Formel (I) jeweils unabhängig voneinander gilt:
R₁ bedeutet Wasserstoff, Methyl, Acetyl oder Formyl, vorzugsweise Wasserstoff oder Methyl,
R₂ bedeutet Methyl, Formyl oder Acetyl, vorzugsweise Formyl oder Acetyl,
R₃ bedeutet Wasserstoff oder Methyl und
R₄ bedeutet Methyl, Ethyl, Propyl, Isopropyl, Butyl, sec-Butyl, tert-Butyl oder iso-Butyl, vorzugsweise Methyl, Ethyl, Isopropyl oder tert-Butyl.

Die nachfolgenden Verbindungen 1 bis 8 sind für die Zwecke der vorliegenden Erfindung besonders geeignet:

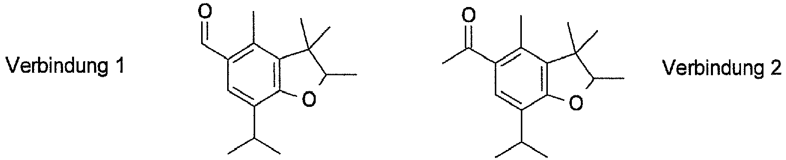

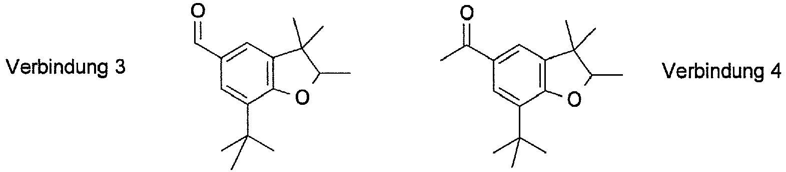

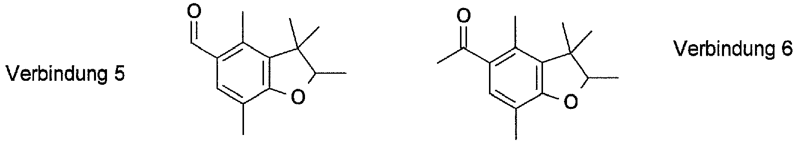

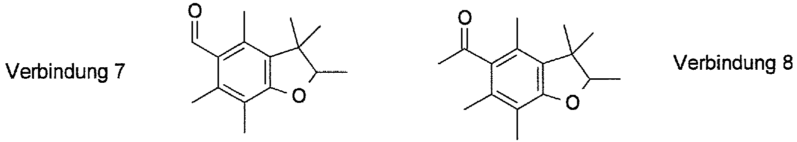

Dementsprechend ist eine Verwendung (wie oben beschrieben) besonders bevorzugt, wobei die Verbindung der Formel (I) bzw. eine, mehrere oder sämtliche Verbindungen der Formel (I) ausgewählt ist bzw. jeweils unabhängig voneinander ausgewählt sind aus der Gruppe bestehend aus den Verbindungen 1 bis 8, insbesondere aus den Verbindungen 2, 4 und 5.

Die Herstellung der erfindungsgemäß zu verwendenden Verbindungen der Formel (I) kann mittels an sich bekannter Reaktionen und Verfahren erfolgen (siehe hierzu auch die beigefügten Beispiele). Beispielsweise können zunächst die entsprechenden Phenole und Kresole mit Allylchlorid oder Allylbromid unter basischen Bedingungen in guten Ausbeuten zu den entsprechenden Prenylethern umgesetzt werden.

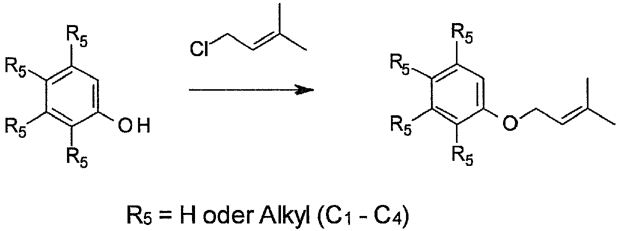

Anschließend können die entsprechenden Prenylether zum Beispiel bei 210°C und mit einer katalytischen Menge an Base zu den erfindungsgemäß zu verwendenden Verbindungen der Formel (I) umgesetzt werden.

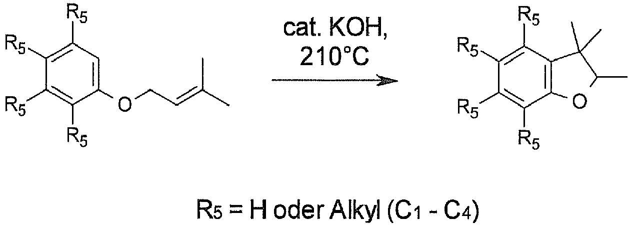

Eine ähnliche Umsetzung ist bereits in Kabalka et al., Journal of Chemical Research 2009, 120-123, beschrieben. Dort wird die Umlagerung und anschließende Cyclisierung von Prenylphenylethern in Gegenwart von Aluminiumoxid und unter Einsatz von Mikrowellen zu 2,3,3-Trimethyldihydrobenzofuranen beschrieben. Anschließend werden die Trimethyldihydrobenzofurane unter Standardbedingungen acetyliert oder formyliert.

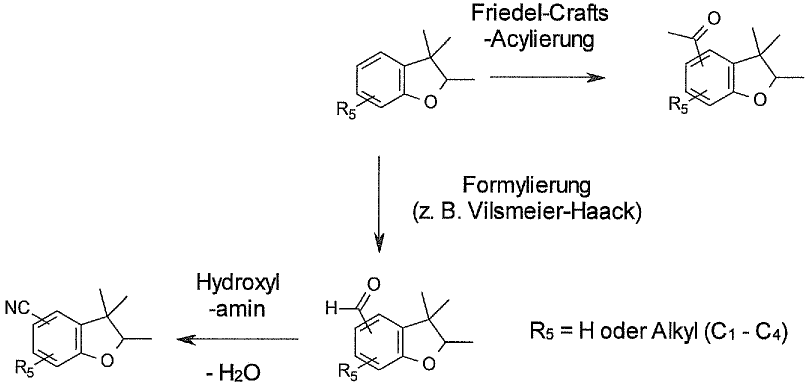

Die formylierten Verbindungen können im Rahmen der vorliegenden Erfindung zu erfindungsgemäß bevorzugten Nitrilen umgesetzt werden.

Für weitere exemplarische Ausführungen bezüglich der Herstellung erfindungsgemäß zu verwendender Verbindungen der Formel (I) verweisen wir auf die beigefügten Beispiele.

Ein weiterer Aspekt der vorliegenden Erfindung betrifft erfindungsgemäße Riechstoff- und/oder Aromastoffmischungen.

Dementsprechend wird im Rahmen der vorliegenden Erfindung auch eine Riechstoff- und/oder Aromastoffmischung, vorzugsweise eine Riechstoffmischung, bereitgestellt, umfassend oder bestehend aus
(a) einer Verbindung der Formel (I) oder einer Mischung aus zwei, drei, vier, fünf, sechs oder mehreren verschiedenen Verbindungen der Formel (I), wie jeweils oben definiert, vorzugsweise wie oben als bevorzugt bezeichnet,
   und
(b) einem, zwei, drei oder mehreren weiteren Riech- und/oder Aromastoffen, wobei der bzw. die weiteren Riech- bzw. Aromastoffe keine Verbindungen der Formel (I) sind.

Für die darin enthaltenen Verbindungen der Formel (I) bzw. entsprechende Mischungen davon gilt dabei jeweils das oben Gesagte entsprechend. Dementsprechend enthält eine erfindungsgemäße Riechstoff- und/oder Aromastoffmischung vorzugsweise solche Verbindungen der Formel (I) bzw. Mischungen davon, die hierin als bevorzugt bezeichnet sind.

Die Verbindung(en) der Formel (I) ist bzw. sind üblicherweise in sensorisch wirksamen Mengen enthalten.

Erfindungsgemäß besonders bevorzugt ist eine Riechstoff- und/oder Aromastoffmischung (wie vorstehend beschrieben), wobei der bzw. ein, zwei, drei, mehr oder sämtliche der weiteren Riech- bzw. Aromastoffe gemäß Bestandteil (b) einen holzigen, einen moschusartigen und/oder einen blumigen Geruch und/oder Geschmack vermitteln.

Gemäß einem bevorzugten Aspekt der vorliegenden Erfindung ist der bzw. ein oder sind zwei, drei, mehr oder sämtliche der weiteren Riech- bzw. Aromastoffe gemäß Bestandteil (b) Riechstoffe mit holzigem Geruch bzw. mit holziger Note (inkl. Sandel und Ambra) oder Riechstoffe mit blumigem Geruch bzw. blumiger Note.

Besonders geeignete holzige Riechstoffe sind: Sandranol (2-Ethyl-4-(2,2,3)-trimethylcyclopent-3-yl-but-2-en-1-ol), Ysamber K (1',1',5',5'-Tetramethylhexahydro-spiro[1,3-dioxolan-2,8'(5'H)-2H-2,4a-methanonaphthalen]), Timberol (1-(2,2,6-Trimethylcyclohexyl)hexan-3-ol), Iso-E-Super, (2,3,8,8,-Tetramethyl-1,2,3,4,5,6,8-octahydro-2-naphthalenyl-methyl-keton), Isobornylacetat (2-exo-Bornanylacetat), Ylanat (2-tert-Butylcyclohexyl-acetat).

Besonders geeignete blumige Riechstoffe sind: Lilial (2-Methyl-3-(4-tert-butylphenyl)propanal), Hedion (Methyl (3-oxo-2-pentyl-cyclopentyl)acetat), Mayol (4-Isopropyl-cyclohexyl) methanol), Linalool (3,7 -Dimethyl-1,6-octadien-3-ol), Dihydromyrcenol (2,6-Dimethyl-7-octen-2-ol), Citronellol (3,7 -Dimethyl-6-octen-1-ol), Phenoxanol (3-Methyl-5-Phenyl-pentanol), 2-Phenylethylalkohol, Hydroxycitronellal (3,7 -Dimethyl-7-hydroxyoctan-1-al), alpha-Jonon (4-(2,6,6-Trimethyl-cyclohex-2-enyl)-but-3-en-2-on).

Die Kombination von Verbindungen der Formel (I) mit oben genannten bevorzugten holzigen Riechstoffen führt zu helleren, strahlenden und sauberen Geruchseindrücken. Die Mischungen wirken vorteilhafterweise natürlicher und frischer.

Die Kombination von Verbindungen der Formel (I) mit oben genannten bevorzugten blumigen Riechstoffen führt zu besonders vorteilhafter Frische und Ausstrahlung. Weiterhin werden die blumigen Aspekte durch Kombination mit einer oder mehreren Verbindungen der Formel (I) als abgerundeter empfunden. Entsprechende Mischungen riechen intensiver und harmonischer.

Gemäß einem weiteren, bevorzugten Aspekt der vorliegenden Erfindung ist der bzw. ein (weiterer) oder sind zwei, drei, mehr (weitere) oder sämtliche der weiteren Riech- bzw. Aromastoffe gemäß Bestandteil (b) grün-fruchtige Riechstoffe. Bevorzugte grün-fruchtige Riechstoffe sind: Vertral (Octahydro-1 H-4,7-methanoinden-5-carbaldehyd), cis-3-Hexen-1-ol, beta-Damascon (1-(2,6,6-Trimethyl-cyclohex-2-enyl)-buten-1-on), Vertocitral (2,4-Dimethylcyclohex-3-en-1-carbaldehyd), Cyclogalbanat (Allyl (Cyclohexyloxy- acetat), Hexylacetat.

Die Kombination dieser Riechstoffe mit Verbindungen der Formel (I) führt vor allem zu einem runderen und weicheren Geruch. Zudem verleiht der Zusatz einer oder mehrerer Verbindungen der Formel (I) einen gewissen Glanz und vermittelt einen Eindruck von natürlicher Ausstrahlung.

Gemäß einem weiteren, bevorzugten Aspekt der vorliegenden Erfindung ist der bzw. ein (weiterer) oder sind zwei, drei, mehr (weitere) oder sämtliche der weiteren Riech- bzw. Aromastoffe gemäß Bestandteil (b) würzig-balsamische Riechstoffe. Bevorzugte würzig-balsamische Riechstoffe sind: Eugenol (2-Methoxy-4-allylphenol), Cumarin (2H-1-Benzopyran-2-on), Anisaldehyd (4-Methoxybenzaldehyd), Amylzimtaldehyd (2-Penyl-3-phenyl-2-propenal), Isoamyisalicylat (Salicylsäure-3-methylbutylester) und Zimtalkohol (3-Phenyl-2-propen-1-ol).

Die Kombination dieser Riechstoffe mit Verbindungen der Formel (I) führt zu besonderer Frische und Natürlichkeit. Die Mischungen wirken harmonischer und strahlender.

In Mischungen mit weiteren Riechstoffen vermögen die Verbindungen der Formel (I) vorteilhafterweise bereits in geringen Dosierungen die Intensität einer Riechstoffmischung zu verstärken und das Gesamtbild der Riechstoffmischung vor allem geruchlich abzurunden und zu harmonisieren sowie der Mischung mehr (Aus-)Strahlung und Natürlichkeit zu verleihen.

Für eine erfindungsgemäße Riechstoff- und/oder Aromastoffmischung (wie oben beschrieben) gilt gemäß einer bevorzugten Ausgestaltung, dass
- die Gesamtmenge an Verbindungen der Formel (I) im Bereich von 0,00001 bis 99,9 Gew.-%, vorzugsweise im Bereich von 0,001 bis 70 Gew.-%, besonders bevorzugt im Bereich von 0,01 bis 50 Gew.-%, liegt, bezogen auf das Gesamtgewicht der Riechstoff- bzw. Aromastoffmischung,
   und/oder
- das Gewichtsverhältnis der Gesamtmenge an Bestandteil (a) zur Gesamtmenge an Bestandteil (b) in der Riechstoff- bzw. Aromastoffmischung im Bereich von 1 : 100.000 bis 10 : 1 liegt, vorzugsweise im Bereich von 1 : 10.000 bis 5 : 1, insbesondere im Bereich von 1 : 1.000 bis 2 : 1.

Insbesondere für den Fall, dass die erfindungsgemäß zu verwendenden Verbindungen der Formel (I) bzw. eine entsprechende Mischung eingesetzt werden soll bzw. wird, um einer Riechstoff- und/oder Aromastoffmischung bzw. -komposition (wie weiter unten beschrieben) mehr (Aus-)Strahlung, Abrundung und/oder Harmonie zu verleihen und/oder bestimmte Noten zu verstärken, liegt die Gesamtmenge an Bestandteil (a) gemäß einer bevorzugten Ausgestaltung der vorliegenden Erfindung im Bereich von 0,01 bis 5 Gew.-%, bevorzugt im Bereich von 0,1 bis 2 Gew.-%, bezogen auf das Gesamtgewicht der Mischung bzw. Komposition.

Je nach gewünschter Anwendung kann es vorteilhaft und demnach erfindungsgemäß bevorzugt sein, die Gesamtmenge an Bestandteil (a) so zu wählen, dass in Abhängigkeit von den weiteren Bestandteilen der jeweiligen Mischung bzw. Komposition (noch) keine Vermittlung der oben angegebenen Eigengeruchs- und/oder -geschmacksnoten der Verbindung(en) der Formel (I) erreicht wird, jedoch vorzugsweise eine, mehrere oder sämtliche der übrigen positiven Eigenschaften (wie hierin beschrieben) zum Tragen kommen.

Die erfindungsgemäß zu verwendenden Verbindungen der Formel (I) bzw. entsprechende Mischungen davon oder eine erfindungsgemäße Riechstoff- und/oder Aromastoffmischung (wie oben beschrieben) können vorteilhafterweise auch zur Parfümierung und/oder Aromatisierung bestimmter Artikel eingesetzt werden bzw. Bestandteil solcher Artikel sein. Insbesondere eine erfindungsgemäße Riechstoff- und/oder Aromastoffmischung, vorzugsweise eine Riechstoff- und/oder Aromastoffmischung wie hierin als bevorzugt bezeichnet, ist im Rahmen der vorliegenden Erfindung als Bestandteil eines parfümierten und/oder aromatisierten Artikels einsetzbar.

Dementsprechend betrifft die vorliegende Erfindung auch einen parfümierten und/oder aromatisierten Artikel, der eine erfindungsgemäße Riechstoff- und/oder Aromastoffmischung (wie hierin beschrieben) umfasst.

In einem erfindungsgemäßen Artikel ist vorzugsweise eine Gesamtmenge an Verbindung(en) der Formel (I) enthalten, die ausreicht, um eine Geruchs- und/oder Geschmacksnote des Typs moschus, insbesondere strahlend-moschusartig, exaltierend und/oder (weich) holzig zu vermitteln, zu modifizieren und/oder zu verstärken. Besonders bevorzugt ist die Gesamtmenge an Verbindung(en) der Formel (I) ausreichend, um einen, mehrere oder sämtliche der hierin beschriebenen, durch die erfindungsgemäß zu verwendenden Verbindungen bzw. Mischungen zu erreichenden Vorteile zu erzielen.

Hinsichtlich der bevorzugt enthaltenen Verbindungen der Formel (I) bzw. Mischungen davon sowie die bevorzugt enthaltenen weiteren Riechstoffe gilt das oben Gesagte entsprechend. Ein erfindungsgemäßer Artikel umfasst demnach vorzugsweise eine erfindungsgemäße Riechstoff- und/oder Aromastoffmischung, die hierin als erfindungsgemäß bevorzugt bezeichnet wird. Die (enthaltene) erfindungsgemäße Riechstoff- und/oder Aromastoffmischung umfasst bzw. besteht wiederum vorzugsweise aus solchen Riech- und/oder Aromastoffen, die hierin als erfindungsgemäß bevorzugte Verbindungen der Formel (I) bzw. als bevorzugt zu verwendende weitere Riechstoffe (siehe insbesondere Bestandteil (b)) bezeichnet werden. Das oben bezüglich bevorzugter Mengen bzw. Gewichtsverhältnisse Gesagte gilt hierbei entsprechend.

Gemäß einer Ausgestaltung der vorliegenden Erfindung ist ein erfindungsgemäßer Artikel eine Riechstoff- und/oder Aromastoffkomposition.

Bevorzugte Substanzen, die im Rahmen der vorliegenden Erfindung in einem erfindungsgemäßen Artikel, einer erfindungsgemäßen Riechstoff- und/oder Aromastoffkomposition oder in einer erfindungsgemäßen Riechstoff- und/oder Aromastoffmischung als weitere Substanzen, vorzugsweise als weitere Riechstoffe (neben Verbindungen der Formel (I)), enthalten sind bzw. enthalten sein können, finden sich z. B. in S. Arctander, Perfume and Flavor Materials, Vol. I und II, Montclair, N. J. 1969, Eigenverlag, oder K. Bauer et al., Common Fragrance and Flavor Materials, 4th Edition, Wiley-VCH, Weinheim 2001.

Im Einzelnen seien genannt:
Extrakte aus natürlichen Rohstoffen wie Etherische Öle, Concretes, Absolues, Resine, Resinoide, Balsame und Tinkturen, z. B.
Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepreßt; Linaloeöl; Litseacubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierte Inhaltsstoffe;
Einzel-Riechstoffe aus der Gruppe
der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; 1-Octen-3-ol; Gemisch von 3,4,5,6,6-Pentamethyl-314-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecena1;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercapto hexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadien-oat;4-Methyl-2-pentyl-crotonat;
der acyclischen Terpenalkohole wie z. B. Geraniol; Nerol; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-oi 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Citronellal;; 7-Methoxy-3,7-dimethyloctanal; 2,6,10-Trimethyl-9-undecenal; Geranylaceton; sowie die Dimethyl- und Diethylacetale von Geranial, Neral,
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethyl-ionon; alpha-Iron; beta-Damascenon; 1-(2,4,4-Trimethyl-2-cyclohexen-1-yl)-2-buten-1-on; 1,3,4,6,7,8a-Hexahydro-1,1,5,5-tetramethyl-2H-2,4a-methano-naphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclo-dodecan; alpha-Cedrenepoxid; 3a,6,6,9a-Tetramethyldodecahydro-naphtho[2,1-b]furan; 3a-Ethyl-6,6,9a-trimethyldodecahydronaphtho[2,1-b]furan; 1,5,9-Tri-methyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentyl-cyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopenta-decanon; 4-(1 -Ethoxyvinyl)-3,3,5,5-tetramethylcyclohexanon; 4-tert.-Pentylcyclo-hexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentyl-cyclo-hexyl-acetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexa-hydro-5, bzw. 6-indenylacetat; 4,7-Methano-3a,4,5,6,7,7a-hexahydro-5, bzw. 6-indenyl-propionat; 4,7-Methano-3a,4,5,6,7,7a-hexahydro-5, bzw. 6-inden-ylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexen-carboxylat; Ethyl-2-methyl-1,3-dioxolan-2-acetat;
der araliphatischen Alkohole wie z.B. Benzylalkohol; 1-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenyl-propanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenyl-ethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethyl-isoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetra-hydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-isobutylphenyl)propanal; 3-(4-tert.-Butyl-phenyl)propanal; Zimtaldehyd; alpha-Butylzimtaldehyd; alpha-Hexylzimtaldehyd; 3-Methyl-5-phenylpentanal; 4-Methoxybenzaldehyd; 4-Hydroxy-3-methoxybenz-aldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylaceto-phenon; 4-Phenyl-2-butanon; 4-(4-Hydroxyphenyl)-2-butanon; 1-(2-Naphtha-lenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-di-methyl-4-indanylmethylketon; 1-[2,3-dihydro-1,1,2,6-tetramethyl-3-(1-methylethyl) -1H-5-indenyl]ethanon; 5',6',7',8'-Tetrahydro-3',5',5',6',8',8'-hexamethyl-2-aceto-naphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentan-säurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butyl-phenyl)propanal oder 2,4-Dimethyl-3-cyclohexencarbaldehyd; 6-Isopropyl-chinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenylmethylether; Isoeugenol; Isoeugenylmethylether; Thymol; Carvacrol; Diphenylether; beta-Naphthylmethylether; beta-Naphthylethylether; beta-Naphthyliso-butylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; 1,16-Hexadeca-nolid; 9-Hexadecen-1,16-olid; 10-Oxa-1,16-hexadecanolid; 11-Oxa-1,16-hexa-decanolid; 12-Oxa-1,16-hexadecanolid; Ethylen-1,12-dodecandioat; Ethylen-1,13-tridecandioat; 2,3-Dihydrocumarin; Octahydrocumarin.

Eine erfindungsgemäße Riechstoff- und/oder Aromastoffmischung oder eine erfindungsgemäße Riechstoff- und/oder Aromastoffkomposition bzw. ein erfindungsgemäßer Artikel wird gemäß einer bevorzugten Ausgestaltung hergestellt, indem Bestandteil (a) (wie oben beschrieben) oder eine Mischung von Bestandteil (a) mit einem (optionalen) weiteren Bestandteil (c) sowie gegebenenfalls weiteren Bestandteilen, bestehend aus gegebenenfalls in einer erfindungsgemäßen Mischung bzw. Komposition zusätzlich enthaltenen Substanzen, bereitgestellt wird und mit einem oder mehreren weiteren Riech- und/oder Aromastoffen (siehe Bestandteil (b)) gemischt wird. Bestandteil (a) wird dabei vorzugsweise in einer Gesamtmenge eingesetzt, die ausreicht, um in der fertigen Mischung oder Komposition bzw. in dem fertigen Artikel einen Geruchs- und/oder eine Geschmacksnote des oben beschriebenen Typs zu vermitteln, zu modifizieren und/oder zu verstärken. Auch im Übrigen gilt für bevorzugte Mengen und Gewichtsverhältnisse das oben Gesagte entsprechend.

Erfindungsgemäße Riechstoff- und/oder Aromastoffkompositionen oder -mischungen können in flüssiger Form, unverdünnt oder mit einem Lösungsmittel verdünnt vorliegen und gegebenenfalls zur Parfümierung oder Aromatisierung eingesetzt werden. Geeignete Lösungsmittel hierfür sind z.B. Ethanol, Isopropanol, Diethylenglycolmonoethyleter, Glycerin, Propylenglycol, 1,2-Butylenglycol, Dipropylenglycol, Diethylphtalat, Triethylcitrat, Isopropylmyristat und Triacetin.

Des Weiteren können erfindungsgemäße Riechstoff- und/oder Aromastoffkompositionen oder -mischungen an einem Trägerstoff adsorbiert sein, der sowohl für eine feine Verteilung der Riech- und/oder Aromastoffe im Produkt, als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein. Die Kombination aus erfindungsgemäßer Komposition oder -mischung und Trägerstoff stellt einen (beispielhaften) erfindungsgemäßen Artikel dar.

Insbesondere erfindungsgemäße Riechstoff- und/oder Aromastoffkompositionen können auch mikroverkapselt, sprühgetrocknet, als Einschluss-Komplexe oder als Extrusions-Produkte vorliegen und in dieser Form gegebenenfalls wiederum einem (weiteren) zu parfümierenden und/oder aromatisierenden Produkt bzw. Artikel hinzugefügt werden.

Gegebenenfalls können die Eigenschaften der erfindungsgemäßen Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Artikel dar.

Die Mikroverkapselung der erfindungsgemäßen Riechstoff- und/oder Aromastoffkompositionen kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Riechstoff- und/oder Aromastoffkompositionen können beispielsweise durch Sprühtrocknung einer die Riechstoff- und/oder Aromastoffkomposition enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riech- oder Aromastoffkomposition und Cyclodextrinen oder Harnstoffderivaten in ein geeignetes Lösungsmittel, z.B. Wasser, hergestellt werden. Extrusions-Produkte können durch Verschmelzen der Riechstoff- und/oder Aromastoffkompositionen mit einem geeigneten wachsartigen Stoff und durch Extrusion mit nachfolgender Erstarrung, ggf. in einem geeigneten Lösungsmittel, z.B. Isopropanol, erhalten werden.

Riechstoff- und/oder Aromastoffkompositionen oder Riechstoff- und/oder Aromastoffmischungen, wie jeweils hierin beschrieben, können in konzentrierter Form, in Lösungen oder in oben beschriebener modifizierter Form verwendet werden, insbesondere für die Herstellung von erfindungsgemäßen (parfümierten) Artikeln, z. B. Parfüm-Extraits, Eau de Parfums, Eau de Toilettes, Rasierwässer, Eau de Colognes, Pre-shave-Produkte, Splash-Colognes und parfümierten Erfrischungstüchern, sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und - lotionen, Bräunungscremes und -lotionen, Haarpflegeprodukten wie z.B. Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien und Treibstoffen.

Dementsprechend ist ein erfindungsgemäßer Artikel (wie hierin beschrieben) vorzugsweise ausgewählt aus der Gruppe bestehend aus: Parfüm-Extraits, Eau de Parfums, Eau de Toilettes, Rasierwässer, Eau de Colognes, Pre-shave-Produkte, Splash-Colognes, parfümierte Erfrischungstüchern, saure, alkalische und neutrale Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und - lotionen, Enthaarungscremes und -lotionen, After-shave-Cremes und -lotionen, Bräunungscremes und -lotionen, Haarpflegeprodukten, Deodorantien und Antiperspirantien, Produkten der dekorativen Kosmetik, Kerzen, Lampenölen, Räucherstäbchen, Insektiziden, Repellentien und Treibstoffen.

Die erfindungsgemäß zu verwendenden Verbindungen der Formel (I) bzw. entsprechende Mischungen davon, erfindungsgemäße Riechstoff- und/oder Aromastoffmischungen oder erfindungsgemäße Riechstoff- und/oder Aromastoffkompositionen können (wiederum) in (weitere) aromatisierte oder zu aromatisierende Artikel eingearbeitet werden, insbesondere in der Ernährung, der Mundpflege oder dem Genuss dienende Zubereitungen. Solche Zubereitungen sind wiederum erfindungsgemäße Artikel (wie hierin beschrieben).

Der Ernährung oder dem Genuss dienende Zubereitungen sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Nach Einarbeiten der erfindungsgemäß zu verwendenden Verbindungen, der entsprechenden Mischungen, erfindungsgemäßer Riechstoff- und/oder Aromastoffmischungen oder erfindungsgemäßer Riechstoff- und/oder Aromastoffkompositionen sind diese Zubereitungen erfindungsgemäße Zubereitungen (als bevorzugte Beispiele erfindungsgemäßer Artikel).

Erfindungsgemäße Zubereitungen können z. B. als Halbfertigware oder als Würzmischung vorliegen.

Erfindungsgemäße Zubereitungen können insbesondere als Halbfertigware zur Herstellung weiterer der Ernährung oder dem Genuss dienenden Zubereitungen dienen, insbesondere in sprühgetrockneter Form. Erfindungsgemäße Zubereitungen können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende erfindungsgemäße Zubereitungen sind insbesondere Mund- und/oder Zahnpflegemittel wie Zahnpasten, Zahngele, Zahnpulver, Mundwässer, Kaugummis und andere Mundpflegemittel.

Weitere übliche Wirk-, Grund-, Hilfs- und Zusatzstoffe für der Ernährung, der Mundpflege oder dem Genuss dienende erfindungsgemäße Zubereitungen können in Mengen von 5 bis 99,999999 Gew.-%, vorzugsweise 10 bis 80 Gew.-%, bezogen auf das Gesamtgewicht der Zubereitung, enthalten sein. Ferner können die Zubereitungen Wasser in einer Menge bis zu 99,999999 Gew.-%, vorzugsweise 5 bis 80 Gew.-%, bezogen auf das Gesamtgewicht der Zubereitung, aufweisen.

Erfindungsgemäße Zubereitungen werden gemäß einer bevorzugten Ausgestaltung hergestellt, indem eine erfindungsgemäß zu verwendende Verbindung der Formel (I) oder eine Mischung verschiedener Verbindungen der Formel (I) (wie hierin beschrieben) bzw. vorstehend beschriebene Mischungen bzw. Kompositionen als Substanz, als Lösung (z. B. in Ethanol, Wasser oder 1,2-Propylenglycol) oder in Form eines Gemisches mit einem festen oder flüssigen Trägerstoff (z.B. Maltodextrin, Stärke, Silicagel), gegebenenfalls mit sonstigen Aromen bzw. Aromastoffen und/oder Riechstoffen sowie gegebenfalls (weiteren) Hilfsmitteln und/oder Stabilisatoren (z.B. natürliche oder künstliche Polysaccharide und/oder Pflanzengummen wie modifizierte Stärken oder Gummi Arabicum) in eine der Ernährung, der Mundpflege oder dem Genuss dienende Basis-Zubereitung eingearbeitet werden. Vorteilhafterweise können als Lösung und/oder Suspension oder Emulsion vorliegende erfindungsgemäße Zubereitungen auch durch Sprühtrocknung in eine feste erfindungsgemäße Zubereitung (Halbfertigware) überführt werden.

Die erfindungsgemäßen sprühgetrockneten festen Zubereitungen (als Beispiel erfindungsgemäßer Artikel) sind als Halbfertigwaren besonders gut zur Herstellung von weiteren erfindungsgemäßen Zubereitungen geeignet. In den erfindungsgemäßen sprühgetrockneten festen Zubereitungen sind vorzugsweise 50 bis 95 % Gew.-% Trägerstoffe, insbesondere Maltodextrin und/oder Stärke, 5 bis 40 % Hilfsstoffe, bevorzugt natürliche oder künstliche Polysaccharide und/oder Pflanzengummen wie modifizierte Stärken oder Gummi Arabicum enthalten.

Gemäß einer weiteren bevorzugten Ausführungsform werden zur Herstellung erfindungsgemäßer Zubereitungen eine oder mehrere Verbindungen der Formel (I) (wie oben beschrieben) sowie gegebenenfalls weitere Bestandteile (vorzugsweise wie oben beschrieben) der erfindungsgemäßen Zubereitung zunächst in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten (z.B. modifizierte Stärke), Cellulose oder Cellulosederivaten (z.B. Hydroxypropylcellulose), anderen Polysacchariden (z.B. Dextrin, Alginat, Curdlan, Carageenan, Chitin, Chitosan, Pullulan), natürlichen Fetten, natürlichen Wachsen (z.B. Bienenwachs, Carnaubawachs), aus Proteinen, z.B. Gelatineoder sonstigen Naturprodukten (z.B. Schellack) eingearbeitet. Dabei können je nach Matrix die Produkte durch Sprühtrocknung, Sprühgranulation, Schmelzgranulation, Koazervation, Koagulation, Extrusion, Schmelzextrusion, Emulsionsverfahren, Beschichtung (Coating) oder andere geeignete Verkapselungsverfahren und gegebenenfalls eine geeignete Kombination der vorgenannten Verfahren erhalten werden.

In einem weiteren bevorzugten Herstellungsverfahren für eine erfindungsgemäße Zubereitung werden eine oder mehrere Verbindungen der Formel (I) (wie oben beschrieben) sowie gegebenenfalls weitere Bestandteile zunächst mit einem oder mehreren Komplexbildnern, beispielsweise mit Cyclodextrinen oder Cyclodextrinderivaten, bevorzugt α- oder β-Cyclodextrin, komplexiert und in dieser komplexierten Form eingesetzt.

Besonders bevorzugt ist eine erfindungsgemäße Zubereitung, bei der die Matrix so gewählt ist, dass die Verbindung(en) der Formel (I) sowie gegebenenfalls weitere Riech- und/oder Aromastoffe verzögert von der Matrix freigegeben werden, so dass eine langanhaltende Wirkung erzielt wird. Besonders bevorzugt ist insoweit eine Fett-, Wachs-, Polysaccharid- oder Proteinmatrix.

Als weitere Bestandteile für erfindungsgemäße, der Ernährung oder dem Genuss dienende Zubereitungen können (auch) übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Beispiel für der Mundpflege dienende erfindungsgemäße Zubereitungen) umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin und Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende erfindungsgemäße Zubereitungen) umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkoholen, Geschmackskorrigenzien für unangenehme Geschmackseindrücke, andere Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Bevorzugt können die erfindungsgemäßen Zubereitungen auch eine (weitere) Aromakomposition enthalten, um den Geschmack und/oder Geruch der Zubereitung abzurunden und zu verfeinern. Geeignete (zusätzliche) Aromakompositionen enthalten z.B. synthetische, natürliche oder naturidentische Aroma-, Riech- und Geschmacksstoffe sowie geeignete Hilfs- und Trägerstoffe.

Wie oben beschrieben, werden im Rahmen der vorliegenden Erfindung auch gegenüber dem Stand der Technik neue Verbindungen der Formel (I) sowie entsprechende Mischungen davon bereitgestellt.

Dementsprechend betrifft ein weiterer Aspekt der vorliegenden Erfindung eine Verbindung der Formel (I) 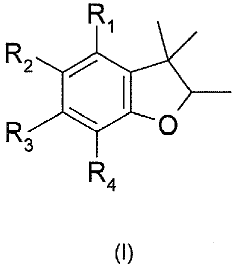 oder eine Mischung bestehend aus zwei, drei, vier, fünf, sechs oder mehreren verschiedenen Verbindungen der Formel (I),
wobei für die Reste der Verbindung der Formel (I) bzw. die Reste sämtlicher Verbindungen der Formel (I) jeweils unabhängig voneinander gilt:
R₁ bedeutet Wasserstoff, Methyl, tert-Butyl, Acetyl, Formyl oder Cyano, vorzugsweise Wasserstoff oder Methyl,
R₂ bedeutet Wasserstoff, Methyl, tert-Butyl, Formyl, Acetyl oder Cyano, vorzugsweise Formyl oder Acetyl,
R₃ bedeutet Wasserstoff, Methyl oder tert-Butyl, und
R₄ bedeutet Wasserstoff, Methyl, Ethyl, Propyl, Isopropyl, Butyl, sec-Butyl, tert-Butyl, iso-Butyl, Formyl, Acetyl oder Cyano, vorzugsweise Methyl, Ethyl, Isopropyl oder tert-Butyl,
vorzugsweise wobei für die Reste der Verbindung der Formel (I) bzw. die Reste sämtlicher Verbindungen der Formel (I) jeweils unabhängig voneinander gilt:
   R₁ bedeutet Wasserstoff, Methyl, Acetyl oder Formyl, vorzugsweise Wasserstoff oder Methyl,
   R₂ bedeutet Methyl, Formyl oder Acetyl, vorzugsweise Formyl oder Acetyl,
   R₃ bedeutet Wasserstoff oder Methyl und
   R₄ bedeutet Methyl, Ethyl, Propyl, Isopropyl, Butyl, sec-Butyl, tert-Butyl oder iso-Butyl, vorzugsweise Methyl, Ethyl, Isopropyl oder tert-Butyl.

Die vorstehend und nachfolgend beschriebenen Verbindungen und Mischungen sind im Rahmen der vorliegenden Erfindung bevorzugt einzusetzende bzw. zu verwendende Verbindungen bzw. Mischungen.

Besonders bevorzugt ist eine Verbindung oder Mischung (wie oben beschrieben), wobei für die Reste der Verbindung der Formel (I) bzw. die Reste sämtlicher Verbindungen der Formel (I) jeweils unabhängig voneinander gilt:
R₁ bedeutet Wasserstoff oder Methyl,
R₂ bedeutet Formyl oder Acetyl,
R₃ bedeutet Wasserstoff oder Methyl und
R₄ bedeutet Methyl, Ethyl, Isopropyl oder tert-Butyl.

Weiter bevorzugt ist eine Verbindung oder Mischung (wie oben beschrieben), wobei die Verbindung der Formel (I) bzw. eine, mehrere oder sämtliche Verbindungen der Formel (I) ausgewählt ist bzw. jeweils unabhängig voneinander ausgewählt sind aus der Gruppe bestehend aus den folgenden Verbindungen 1 bis 8, vorzugsweise aus den Verbindungen 2, 4 und 5:

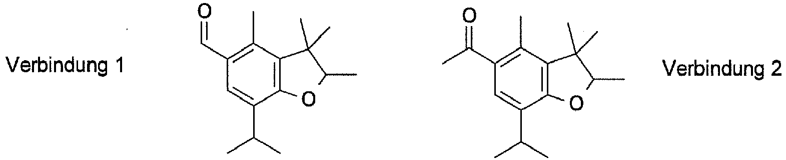

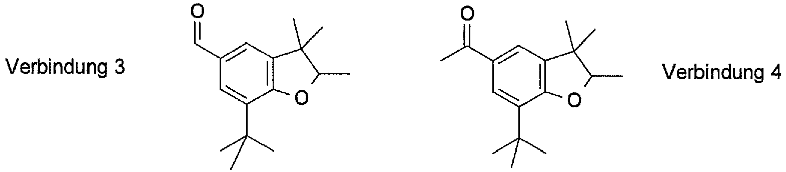

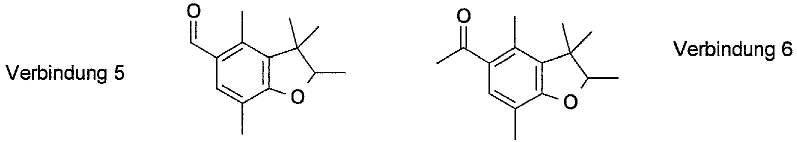

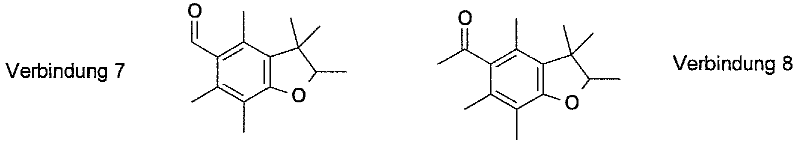

Ein weiterer Aspekt der vorliegenden Erfindung betrifft ein Verfahren zum Vermitteln, Modifizieren und/oder Verstärken eines Geruchs und/oder Geschmacks, vorzugsweise eines moschusartigen und/oder holzigen Geruchs und/oder Geschmacks, umfassend folgenden Schritt:
In Kontakt Bringen oder Mischen einer sensorisch wirksamen Menge
   - einer Verbindung der Formel (I) 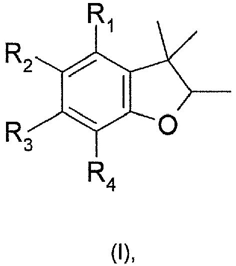 wie oben definiert,
   - einer Mischung aus zwei, drei, vier, fünf, sechs oder mehreren verschiedenen Verbindungen der Formel (I),
      wie oben definiert,
      oder
   - einer erfindungsgemäßen Riechstoff- und/oder Aromastoffmischung (wie hierin beschrieben)
mit einem Erzeugnis.

Bevorzugte Erzeugnisse sind die oben beschriebenen erfindungsgemäß bevorzugten Artikel bzw. Zubereitungen.

Auch im Übrigen gilt für die Auswahl der Verbindungen der Formel (I), die Zusammensetzung einer erfindungsgemäßen Riechstoff- und/oder Aromastoffmischung sowie die bevorzugten Mengen und Gewichtsverhältnisse das oben Gesagte entsprechend.

Im Rahmen der vorliegenden Erfindung wird auch die Verwendung
- einer Verbindung der Formel (I) 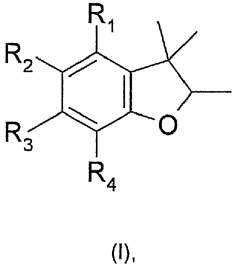 wie oben definiert,
- einer Mischung aus zwei, drei, vier, fünf, sechs oder mehreren verschiedenen Verbindungen der Formel (I),
   wie oben definiert,
   oder
- einer erfindungsgemäßen Riechstoff- und/oder Aromastoffmischung (wie hierin beschrieben)
   oder
- eines erfindungsgemäßen Artikels (wie hierin beschrieben)
   zum Versehen von (a) Haaren, (b) Haut oder (c) textilen Fasern mit einem - insbesondere moschus-artigen - Duft und/oder Geschmack (hinsichtlich weiterer bevorzugter Geruchs- bzw. Geschmacksnoten s. oben) beschrieben.

Ein weiterer Aspekt im Zusammenhang mit der vorliegenden Erfindung betrifft die Verwendung
- einer Verbindung der Formel (I) 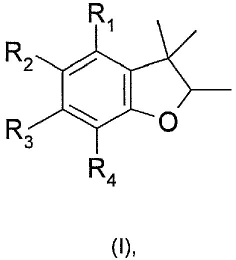 wie oben definiert,
- einer Mischung aus zwei, drei, vier, fünf, sechs oder mehreren verschiedenen Verbindungen der Formel (I),
   wie oben definiert,
   oder
- einer erfindungsgemäßen Riechstoff- und/oder Aromastoffmischung (wie hierin beschrieben)
als Mittel zum Erhöhen der Substantivität und/oder Retention einer Riechstoffmischung und/oder als Fixateur und/oder als Mittel zum Erhöhen des über einer tensidhaltigen wässrigen Lösung wahrgenommenen Geruches anderer Riechstoffe.

Im Zusammenhang mit der Verwendung zum Modifizieren und/oder Verstärken einer Geruchs- und/oder Geschmacksnote steht auch die (erfindungsgemäße) Erkenntnis, dass die erfindungsgemäß zu verwendenden Verbindungen bzw. die entsprechenden Mischungen (wie oben beschrieben) besonders gut als sogenannte Booster (Verstärker; Enhancer) fungieren können. Demnach sind auch entsprechende diesbezügliche Verwendungen von Verbindungen der Formel (I) als zum Gegenstand der vorliegenden Erfindung gehörend zu erachten.

Nachfolgend wird die vorliegende Erfindung anhand von Beispielen näher erläutert. Sofern nicht anders angegeben ist, beziehen sich alle Angaben auf das Gewicht.

### Beispiel 1: Allgemeine Vorschrift zur Herstellung von Prenyl-ethern

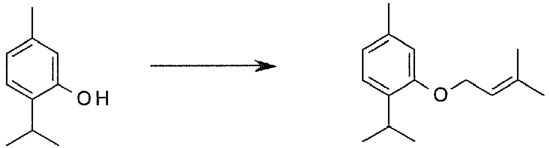

In einen 500 ml Rührwerk werden 60,1 g (0,4 mol) Thymol bei Raumtemperatur zu einer Lösung aus 52,3 g (80%ig, 0,4 mol) Prenylchlorid in 250 ml N-Methyl-2-pyrrolidon (NMP) gegeben. Anschließend werden 35,2 g wässrige NaOH (50%ig, 0,44 mol) langsam unter gleichzeitiger Eiskühlung zugetropft und nach vollendeter Zugabe auf 40°C erhitzt und 2 Stunden Rühren gelassen. Der Ansatz wird dann mit 250 ml Wasser versetzt, mit 2 M NaOH neutralisiert, und die werden Phasen getrennt. Die wässrige Phase wird zweifach mit 150 ml MtBE extrahiert, die organischen Phasen werden vereinigt, getrocknet und eingeengt. Das Rohprodukt (106 g) wird an einer 30 cm Vigreux-Kolonne im Vakuum fraktioniert.

Bei eigener Durchführung wurden 54,1 g in 98 %iger Reinheit erhalten. Dies entspricht einer theoretischen Ausbeute von 61 % (Sdp.: 95°-105°C / 2 mbar).

Entsprechend dieser Vorschrift können weitere Prenylether mit guten bis sehr guten Ausbeuten synthetisiert werden.

### Beispiel 2: Allgemeine Vorschrift zur Herstellung von Dihydrobenzofuranen

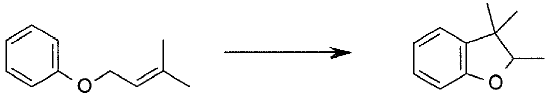

In einem 250 ml Rührwerk werden 121,7 g (0,75 mol) Prenylphenylether mit 0,84 g (15 mmol, 0,02 eq.) gepulvertem KOH und unter stetigem Rühren auf 210°C erhitzt und bei dieser Temperatur 3 h gehalten. Während dieser Zeit werden mittels eines Wasserabscheiders entstehende Leichtsieder abgenommen. Nach Abkühlen auf Raumtemperatur wird der Ansatz mit gesättigter NH₄Cl-Lösung neutralisiert, mit MtBE versetzt, und die Phasen werden getrennt. Die wässrige Phase wird einmal mit MtBE extrahiert. Die vereinigten organischen Phasen werden getrocknet, eingeengt, und das erhaltene Rohprodukt (106,9 g) wird an einer 30 cm Vigreux-Kolonne im Vakuum fraktioniert.

Bei eigener Durchführung wurden 47,9 g in 97 %iger Reinheit erhalten. Dies entspricht einer theoretischen Ausbeute von 38 % (Sdp.: 56°-65°C / 6 mbar).

Entsprechend dieser Vorschrift können weitere Dihydrobenzofurane mit guten bis befriedigenden Ausbeuten synthetisiert werden.

### Beispiel 3: Allgemeine Vorschrift zur Herstellung von Acetyldihydrobenzofuranen

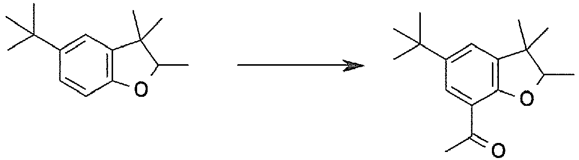

Zu einer Lösung aus 4,00 g (30 mmol, 1,2 eq.) Aluminiumchlorid in 500 ml Dichlormethan werden unter Stickstoff bei 0°C 2,06 g (26,2 mmol, 1,05 eq.) Acetylchlorid gegeben. Anschließend werden 5,46 g 5-*tert*-Butyl-2,3,3-trimethyl-2,3-dihydrobenzofuran bei 0°C zugetropft und drei Stunden nachgerührt. Der Ansatz wird auf Eis gegeben, mit 2 M NaOH neutralisiert, einmal mit Dichlormethan extrahiert, und die vereinigten organischen Phasen werden getrocknet und eingeengt. Das so erhaltene Rohprodukt (6,01 g, 60 %ig) wird mit 10 g Ethanol versetzt, 10 Minuten refluxiert, und die beim Abkühlen ausgefallenen Kristalle werden abgesaugt und im Vakuum getrocknet.

Bei eigener Durchführung wurden 2,00 g in 97 %iger Reinheit erhalten. Dies entspricht einer theoretischen Ausbeute von 31 %.

Entsprechend dieser Vorschrift können weitere Acetyldihydrobenzofurane mit guten bis befriedigenden Ausbeuten synthetisiert werden.

### Beispiel 4: Allgemeine Vorschrift zur Formylierung von Dihydrobenzofuranen

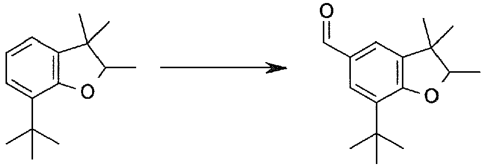

Zu einer Lösung aus 13,5 g (0,1 mol, 2,0 eq.) N-Methylformanilid in 150 ml Toluol werden unter Stickstoff bei Raumtemperatur 12,6 g Phosphorylchlorid (82,5 mmol, 1,6 eq.) zugetropft und eine Stunde bei dieser Temperatur gerührt. Nach Zugabe von 10,9 g (50 mmol) 7-*tert*-Butyl-2,3,3-trimethyl-2H-benzofuran wird auf 80°C erwärmt und vier Stunden bei dieser Temperatur gerührt. Anschließend werden 150 g einer 30%igen Natriumacetatlösung zugegeben und eine Stunde refluxiert. Nach Abkühlen auf Raumtemperatur und der Zugabe von Wasser und MtBE werden die Phasen getrennt, einmal mit MtBE extrahiert, die organischen Phasen vereinigt und getrocknet. Nach Einengen wird das so erhaltene Rohprodukt durch Kugelrohrdestillation von der Sumpf-Fraktion befreit (bis 110°C / 0,1 mbar) und anschließend säulenchromatographisch gereinigt (Kieselgel; Cyclohexan / Essigester = 40/1).

Bei eigener Durchführung wurden 6,13 g in 99 %iger Reinheit erhalten. Dies entspricht einer theoretischen Ausbeute von 50 %.

Entsprechend dieser Vorschrift können weitere formylierte Dihydrobenzofurane mit guten bis befriedigenden Ausbeuten synthetisiert werden.

### Beispiel 5: Allgemeine Vorschrift zur Cyanierung von Formyl-dihydrobenzofuranen

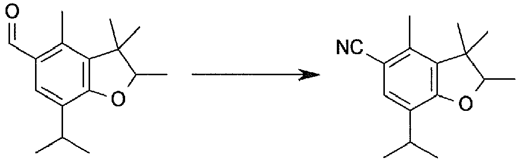

Zu einer Lösung aus 690 mg Hydroxylamin Hydrochlorid (10 mmol) in 40 ml Wasser werden bei Raumtemperatur 800 mg (20 mmol) NaOH und 2,46 g (10 mmol) 7-Isopropyl-2,3,3,4-tetramethyl-2H-benzofuran-5-carbaldehyd gegeben und bei 55°C für 4 h gerührt. Anschließend wird bei Raumtemperatur mit Wasser und MtBE verdünnt, die Phasen getrennt, die wässrige Phase mit MtBE extrahiert, die organischen Phasen vereinigt, getrocknet, eingeengt und das Rohprodukt mit Cyclohexan/Essigester über Kieselgel filtriert. Das so erhaltene Material wird aus 7 g n-Heptan umkristallisiert.

Bei eigener Durchführung wurden so 1,06 g in 97 %iger Reinheit erhalten. Dies entspricht einer theoretischen Ausbeute von 41 %.

Entsprechend dieser Vorschrift können weitere Oxime mit guten bis sehr guten Ausbeuten synthetisiert werden.

Zu einer Lösung aus 8,0 mg Kupfersulfat Pentahydrat (0,031 mmol) in 2,00 g Essigsäureanhydrid werden bei 100°C langsam 1,02 g (3,90 mmol) 7-Isopropyl-2,3,3,4-tetramethyl-2H-benzofuran-5-carbaldehyd oxime (gelöst in 2 g Essigsäureanhydrid) gegeben. Nach 4 h wird der Ansatz auf 0°C abgekühlt, mit Wasser gequencht, mit MtBE versetzt, die Phasen getrennt und die wässrige Phase mit MtBE extrahiert. Die vereinigten organischen Phasen werden getrocknet, eingeengt und das so erhaltene Rohprodukt chromatographisch aufgereinigt (Kieselgel; Cyclohexan / Essigester = 5/1).

Bei eigener Durchführung wurden so 657 mg in 98 %iger Reinheit erhalten. Dies entspricht einer theoretischen Ausbeute von 70 %.

Entsprechend dieser Vorschrift können weitere Nitrilderivate mit guten bis sehr guten Ausbeuten synthetisiert werden.

Die spektroskopischen Daten einiger der im Rahmen von - im Zusammenhang mit der vorliegenden Erfindung - durchgeführten Untersuchungen hergestellten Verbindungen und entsprechenden Intermediaten wurden ermittelt.

Die Daten sind nachfolgend exemplarisch angegeben.

### 1-tert.-Butyl-4-(3-methylbut-2-enoxy)benzene

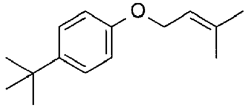

**¹H-NMR (400 MHz, CDCl₃):** δ = 1.30 (s, 9H), 1.75 - 1.72 (m, 3H), 1.81 - 1.77 (m, 3H), 4.49 (d, *J* = 6.8 Hz, 2H), 5.50 (dddd, *J* = 6.8 Hz, *J* = 5.4 Hz, *J* = 2.8 Hz, *J* = 1.4 Hz, 1H), 6.89 - 6.81 (m, 2H), 7.34 - 7.24 (m, 2H) ppm.
**¹³C-NMR (100 MHz, CDCl₃):** δ = 18.16 (q), 25.84 (q), 31.53 (q, q, q), 34.04 (s), 64.66 (t), 114.04 (d, d) 119.93 (d), 126.16 (d, d), 137.96 (s), 143.21 (s), 156.60 (s) ppm.
**MS (EI):** *m*/*z* (%) = 150 (26), 136 (11), 135 (100), 107 (12), 91 (10), 77 (6), 69 (22), 41 (42), 39 (9), 27 (6).

### 5-tert.-Butyl-2,3,3-trimethyl-2H-benzofuran

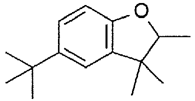

**¹H-NMR (400 MHz, CDCl₃):** δ = 1.09 (s, 3H), 1.30 (s, 9H), 1.31 (s, 3H), 1.36 (d, *J* = 6.6 Hz, 3H), 4.36 (q, *J* = 6.6 Hz, 1H), 6.69 (dd, *J* = 8.3 Hz, *J* = 0.5 Hz, 1H), 7.09 (dd, *J* = 2.2 Hz, *J =* 0.4 Hz, 1 H), 7.12 (dd, *J* = 8.3 Hz, *J* = 2.2 Hz, 1 H) ppm.
**¹³C-NMR (100 MHz, CDCl₃):** δ = 14.41 (q), 23.38 (q), 25.87 (q), 31.77 (q, q, q), 34.38 (s), 43.78 (s), 88.88 (d), 108.61 (d),119.23 (d), 124.47 (d), 137.10 (s), 143.51 (s), 155.96 (s) ppm.
**MS (EI):** *m*/*z* (%) = 218 (23), 204 (16), 203 (100), 147 (15), 91 (9), 69 (7), 57 (30), 43 (8), 41 (25), 29 (10).

### 5-tert.-Butyl-2,2,3-trimethyl-3H-benzofuran

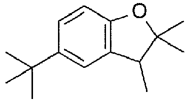

**¹H-NMR (400 MHz, CDCl₃):** δ = 1.23 (d, *J* = 7.2 Hz, 3H), 1.27 (s, 3H), 1.30 (s, 9H), 1.46 (s, 3H), 3.14 (q, *J* = 7.1 Hz, 1 H), 6.62 - 6.65 (m, 1 H), 7.10 - 7.13 (m, 2H) ppm.
**¹³C-NMR (100 MHz, CDCl₃):** δ = 14.73 (q), 22.05 (q), 28.17 (q), 31.77 (q, q, q), 34.28 (s), 45.92 (d), 89.44 (s), 108.54 (d), 120.87 (d), 124.59 (d), 132.32 (s), 142.97 (s), 155.71 (s) ppm.

### 1-(5-tert.-Butyl-2,3,3-trimethyl-2H-benzofuran-7-yl)ethanone

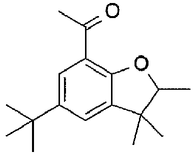

**¹H-NMR (400 MHz, CDCl₃):** δ = 1.12 (s, 3H), 1.31 (s, 9H), 1.33 (s, 3H), 1.41 (d, *J* = 6.6 Hz, 3H), 2.63 (s, 3H), 4.48 (q, *J* = 6.6 Hz, 1H), 7.26 (d, *J* = 2.2 Hz, 1H), 7.71 (d, *J* = 2.2 Hz, 1H) ppm.
**¹³C-NMR (100 MHz, CDCl₃):** δ = 14.71 (q), 23.36 (q), 26.29 (q), 31.15 (q), 31.56 (q, q, q), 34.49 (s), 43.21 (s), 89.91 (d), 119.86 (s), 124.07 (d), 124.58 (d), 139.49 (s), 143.60 (s), 156.86 (s), 197.47 (s) ppm.
**MS (EI):** *m*/*z* (%) = 260 (23), 246 (14), 245 (77), 189 (10), 128 (8), 115 (12), 91 (11), 57 (16), 43 (100), 41 (21).

### 1-tert.-Butyl-2-(3-methylbut-2-enoxy)benzene

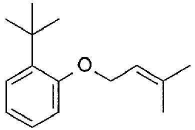

**¹H-NMR (400 MHz, CDCl₃):** δ = 1.38 (s, 9H), 1.72 - 1.74 (m, 3H), 1.77 - 1.81 (m, 3H), 4.54 (d, *J* = 6.5 Hz, 2H), 5.49 - 5.57 (m, 1 H), 6.85 - 6.90 (m, 2H), 7.16 (ddd, *J* = 8.2 Hz, *J* = 7.3 Hz, *J* = 1.7 Hz, 1H), 7.27 (dd, *J =* 8.0 Hz, *J* = 1.8 Hz, 1H) ppm.
**¹³C-NMR (100 MHz, CDCl₃):** δ = 18.23 (q), 25.73 (q), 29.76 (q, q, q), 34.82 (s), 64.85 (t), 112.34 (d), 120.07 (d), 120.35 (d), 126.53 (d), 126.87 (d), 136.65 (s), 138.33 (s), 157.75 (s) ppm.
**MS (EI):** *m*/*z* (%) = 150 (39), 136 (10), 135 (100), 107 (30), 91 (23), 77 (9), 69 (65), 41 (65), 39 (15), 27 (10).

### 7-tert.-Butyl-2,3,3-trimethyl-2H-benzofuran

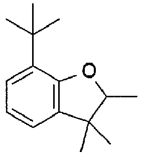

**¹H-NMR (400 MHz, CDCl₃):** δ = 1.08 (s, 3H), 1.28 (s, 3H), 1.35 (d, *J* = 6.6 Hz, 3H), 1.36 (s, 9H), 4.34 (q, *J* = 6.5 Hz, 1 H), 6.81 (dd, *J* = 7.8 Hz, *J* = 7.3 Hz, 1 H), 6.94 (dd, *J* = 7.3 Hz, *J* = 1.4 Hz, 1 H), 7.06 (dd, *J* = 7.8 Hz, *J* = 1.4, 1 H) ppm.
**¹³C-NMR (100 MHz, CDCl₃):** δ = 14.68 (q), 23.05 (q), 26.36 (q), 29.29 (q, q, q), 34.10 (s), 43.18 (s), 87.94 (d), 120.06 (d), 120.11 (d), 124.42 (d), 132.98 (s), 137.91 (s), 156.28 (s) ppm.
**MS (EI):** *m*/*z* (%) = 218 (32), 204 (16), 203 (100), 161 (20), 147 (53), 115 (12), 91 (18), 57 (73), 41 (31), 29 (12).

### 7-tert.-Butyl-2,2,3-trimethyl-3H-benzofuran

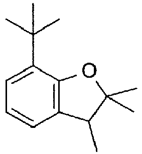

**¹H-NMR (400 MHz, CDCl₃):** δ = 1.20 (d, *J* = 7.1 Hz, 3H), 1.24 (s, 3H), 1.34 (s, 9H), 1.47 (s, 3H), 3.10 (q, *J* = 7.1 Hz, 1H), 6.78 (dd, *J* = 7.8 Hz, *J* = 7.3 Hz, 1H), 6.93 - 6.96 (m, 1 H), 7.05 (dd, *J* = 7.8 Hz, *J* = 1.0 Hz, 1 H) ppm.
**¹³C-NMR (100 MHz, CDCl₃):** δ = 14.53 (q), 21.83 (q), 28.00 (q), 29.18 (q, q, q), 33.99 (s), 45.32 (d), 88.64 (s), 119.57 (d), 121.57 (d), 124.45 (d), 132.83 (s), 133.25 (s), 156.27 (s) ppm.

### 1-(7-tert.-Butyl-2,3,3-trimethyl-2H-benzofuran-5-yl)ethanone

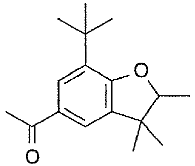

**¹H-NMR (400 MHz, CDCl₃):** δ = 1.12 (s, 3H), 1.32 (s, 3H), 1.38 (s ,9H), 1.38 (d, *J* = 6.5 Hz, 3H), 2.56 (s, 3H), 4.46 (q, *J* = 6.5 Hz, 1 H), 7.60 (d, *J* = 1.9 Hz, 1 H), 7.76 (d, *J* = 1.9 Hz, 1 H) ppm.
**¹³C-NMR (100 MHz, CDCl₃):** ö = 14.80 (q), 23.09 (q), 26.41 (q), 26.59 (q), 29.11 (q, q, q), 34.19 (s), 42.92 (s), 89.46 (d), 121.09 (d), 126.72 (d), 130.50 (s), 132.72 (s), 138.50 (s), 160.87 (s), 197.10 (s) ppm.
**MS (EI):** *m*/*z* (%) = 261 (7), 260 (34), 246 (19), 245 (100), 203 (15), 189 (29), 115 (8), 57 (11), 43 (77), 41 (12).

### 7-tert.-Butyl-2,3,3-trimethyl-2H-benzofuran-5-carbaldehyde

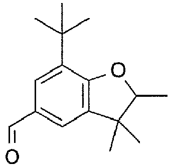

**¹H-NMR (400 MHz, CDCl₃):** δ = 1.13 (s, 3H), 1.33 (s, 3H), 1.38 (s, 9H), 1.38 (d, *J* = 6.6 Hz, 3H), 4.50 (q, *J* = 6.6 Hz, 1H), 7.53 (d, *J* = 1.7 Hz, 1H), 7.61 (d, *J* = 1.7 Hz, 1H), 9.83 (s, 1H) ppm.
**¹³C-NMR (100 MHz, CDCl₃):** δ = 14.82 (q), 23.08 (q), 26.59 (q), 29.05 (q, q, q), 34.18 (s), 42.81 (s), 89.76 (d), 121.67 (d), 129.48 (d), 130.26 (s), 133.52 (s), 139.35 (s), 162.19 (s), 191.27 (d) ppm.
**MS (EI):** *m*/*z* (%) = 246 (36), 232 (17), 231 (100), 189 (17), 175 (34), 147 (13), 115 (9), 91 (10), 57 (19), 41 (10).

### 7-tert.-Butyl-2,3,3-trimethyl-2H-benzofuran-5-carbaldehyde oxime

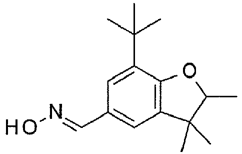

**¹H-NMR (400 MHz, CDCl₃):** δ = 1.10 (s, 3H), 1.30 (s, 3H), 1.34 - 1.37 (m, 12H), 4.40 (q, *J* = 6.5 Hz, 1 H), 7.22 (dd, *J* = 1.8 Hz, *J* = 0.4 Hz, 1 H), 7.23 (dd, *J* = 1.8 Hz, *J* = 0.4 Hz, 1H), 7.61 (s, 1H), 8.05 - 8.13 (m, 1H) ppm.
**¹³C-NMR (100 MHz, CDCl₃):** δ = 14.73 (q), 23.02 (q), 26.42 (q), 29.14 (q, q, q), 34.12 (s), 43.11 (s), 88.80 (d), 118.64 (d), 124.23 (s), 124.93 (d), 133.27 (s), 138.91 (s), 151.02 (d), 158.25 (s) ppm.
**MS (EI):** *m*/*z* (%) = 261 (59), 247 (16), 246 (100), 230 (15), 228 (36), 204 (16), 190 (48), 172 (18), 57 (24), 41 (16).

### 7-tert.-Butyl-2,3,3-trimethyl-2H-benzofuran-5-carbonitrile

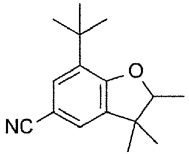

**¹H-NMR (400 MHz, CDCl₃):** δ = 1.11 (s, 3H), 1.30 (s, 3H), 1.34 (s, 9H), 1.37 (d, *J* = 6.6 Hz, 3H), 4.46 (q, *J* = 6.5 Hz, 1 H), 7.21 (d, *J* = 1.7 Hz, 1 H), 7.36 (d, *J* = 1.7 Hz, 1 H) ppm.
**¹³C-NMR (100 MHz, CDCl₃):** δ = 14.69 (q), 23.03 (q), 26.44 (q), 28.89 (q, q, q), 34.31 (s), 43.15 (s), 89.39 (d), 103.19 (s), 120.33 (s), 124.52 (d), 129.98 (d), 134.27 (s), 139.22 (s), 160.09 (s). ppm.
**MS (EI):** *m*/*z* (%) = 243 (27), 229 (17), 228 (100), 186 (17), 173 (6), 172 (48), 158 (9), 57 (10), 41 (6), 28 (10).

### 1-tert.-Butyl-3-(3-methylbut-2-enoxy)benzene

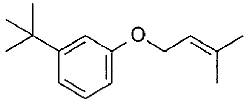

**¹H-NMR (400 MHz, CDCl₃):** δ = 1.30 (s, 9H), 1.72 - 1.75 (m, 3H), 1.78 - 1.82 (m, 3H), 4.50 (dt, *J* = 6.8 Hz, *J* = 0.8 Hz, 2H), 5.45 - 5.57 (m, 1H), 6.73 (ddd, *J* = 8.2 Hz, *J* = 2.4 Hz, *J* = 1.1 Hz, 1 H), 6.93 - 7.01 (m, 2H), 7.17 - 7.26 (m, 1 H) ppm.
**¹³C-NMR (100 MHz, CDCl₃):** δ = 18.18 (q), 25.85 (q), 31.31 (q, q, q), 34.73 (s), 64.56 (t), 110.56 (d), 112.85 (d), 117.75 (d), 119.88 (d), 128.87 (d), 138.02 (s), 152.89 (s), 158.67 (s) ppm.
**MS (EI):** *m*/*z* (%) = 151 (5), 150 (50), 136 (10), 135 (100), 107 (18), 95 (5), 91 (7), 69 (31), 41 (31), 39 (5).

### 6-tert.-Butyl-2,3,3-trimethyl-2H-benzofuran

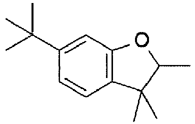

**¹H-NMR (400 MHz, CDCl₃**): δ = 1.08 (s, 3H), 1.29 (s, 3H), 1.29 (s, 9H), 1.36 (d, *J* = 6.6 Hz, 3H), 4.37 (q, *J* = 6.6 Hz, 1H), 6.83 (dd, *J* = 1.7 Hz, *J* = 0.5 Hz, 1H), 6.90 (dd, *J* = 7.8 Hz, *J =* 1.7 Hz, 1H), 7.00 (dd, *J* =7.8 Hz, *J =* 0.5 Hz, 1H) ppm.
**¹³C-NMR (100 MHz, CDCl₃):** δ = 14.47 (q), 23.35 (q), 25.98 (q), 31.50 (q, q, q), 34.75 (s), 43.41 (s), 88.93 (d), 106.87 (d), 117.41 (d), 121.69 (d), 134.53 (s), 151.54 (s), 158.22 (s). ppm.

### 1-(6-tert.-Butyl-2,3,3-trlmethyl-2H-benzofuran-5-yl)ethanone

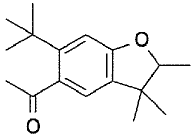

**¹H-NMR (400 MHz, CDCl₃):** δ = 1.09 (s, 3H), 1.30 (s, 3H), 1.35 (s, 9H), 1.37 (d, *J* = 6.6 Hz, 3H), 2.58 (s, 3H), 4.40 (q, *J* = 6.6 Hz, 1H), 6.88 (d, *J* = 0.4 Hz, 1H), 6.90 (d, *J* = 0.3 Hz, 1H) ppm.
**¹³C-NMR (100 MHz, CDCl₃):** δ = 14.51 (q), 23.23 (q), 26.04 (q), 31.88 (q, q, q), 32.48 (q), 36.09 (s), 43.49 (s), 89.48 (d), 108.67 (d), 120.60 (d), 134.08 (s), 135.00 (s), 148.50 (s), 159.09 (s), 207.66 (s) ppm.
**MS (EI):** *m*/*z* (%) = 260 (13), 246 (17), 245 (100), 243 (5), 187 (5), 115 (8), 69 (6), 57 (8), 43 (41), 41 (8).

### 1-Isopropyl-2-(3-methylbut-2-enoxy)benzene

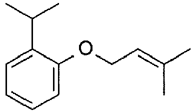

**¹H-NMR (400 MHz, CDCl₃):** ö = 1.21 (d, *J* = 6.9 Hz, 6H), 1.71 - 1.75 (m, 3H), 1.76 - 1.82 (m, 3H), 3.35 (hept., *J* = 7.0 Hz, 1H), 4.52 (d, *J* = 6.4 Hz, 2H), 5.46 - 5.55 (m, 1H), 6.85 (dd, *J* = 8.1 Hz, *J* = 1.2 Hz, 1 H), 6.91 (ddd, *J* = 7.5 Hz, *J* = 1.2 Hz, 1 H), 7.13 (ddd, *J* = 8.2 Hz, *J* = 7.4 Hz, *J* = 1.8 Hz, 1 H), 7.21 (dd, *J* = 7.5 Hz, *J* = 1.8 Hz, 1 H) ppm.
**¹³C-NMR (100 MHz, CDCl₃):** δ = 18.24 (q), 22.73 (q, q), 25.77 (q), 26.70 (d), 65.12 (t), 111.74 (d), 120.39 (d), 120.50 (d), 126.02 (d), 126.39 (d), 137.00 (s), 137.39 (s), 156.07 (s) ppm.
**MS (EI):** *m*/*z* (%) = 136 (47), 122 (9), 121 (100), 91 (23), 77 (12), 69 (52), 65 (9), 41 (60), 39 (15), 27 (10).

### 7-Isopropyl-2,3,3-trimethyl-2H-benzofuran

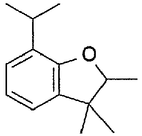

**¹H-NMR (400 MHz, CDCl₃):** δ = 1.09 (s, 3H), 1.24 (dd, *J* = 7.0 Hz, *J* = 0.7 Hz, 6H), 1.30 (s, 3H), 1.36 (d, *J* = 6.6 Hz, 3H), 3.11 (hept., *J* = 6.7 Hz, 1H), 4.34 (q, *J* = 6.6 Hz, 1H), 6.83 (dd, *J* = 7.6 Hz, *J* = 7.3 Hz, 1 H), 6.92 (dd, *J* = 7.3 Hz, *J* = 1.5 Hz, 1 H), 7.01 (ddd, *J* = 7.6 Hz, *J* = 1.5 Hz, *J* = 0.6 Hz, 1 H) ppm.
**¹³C-NMR (100 MHz, CDCl₃):** δ = 14.58 (q), 22.44 (q), 22.48 (q), 23.21 (q), 26.17 (q), 27.89 (d), 43.72 (s), 88.33 (d), 119.72 (d), 120.47 (d), 124.51 (d), 130.61 (s), 137.08 (s), 155.65 (s) ppm.
**MS (EI):** *m*/*z* (%) = 204 (27), 189 (53), 147 (100), 133 (22), 119 (29), 115 (14), 91 (27), 77 (12), 43 (24), 41 (22).

### 7-tert.-Butyl-2,2,3-trimethyl-3H-benzofuran

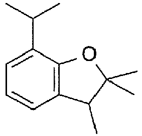

**¹H-NMR (400 MHz, CDCl₃):** δ = 1.19 - 1.26 (m, 12H), 1.47 (d, *J* = 0.5 Hz, 3H), 3.04 (hept., *J* = 6.9 Hz, 1 H), 3.13 (dq, *J* = 7.3 Hz, *J* = 0.9 Hz, 1 H), 6.80 (dd, *J* = 7.6 Hz, *J* = 7.3 Hz, 1H), 6.91 - 6.94 (m, 1H), 6.97 - 7.01 (m, 1H) ppm.
**¹³C-NMR (100 MHz, CDCl₃):** δ = 14.61 (q), 21.94 (q), 22.17 (q), 22.31 (q), 28.04 (q), 28.09 (d), 45.82 (d), 88.86 (s), 119.94 (d), 121.20 (d), 124.61 (d), 130.48 (s), 132.36 (s), 155.59 (s) ppm.

### 1-(7-Isopropyl-2,3,3-trimethyl-2H-benzofuran-5-yl)ethanone

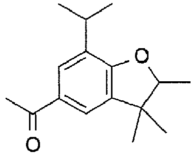

**¹H-NMR (400 MHz, CDCl₃):** δ = 1.12 (s, 3H), 1.27 (dd, *J* = 6.9 Hz, *J* = 0.9 Hz, 6H), 1.33 (s, 3H), 1.38 (d, *J* = 6.5 Hz, 3H), 2.56 (s, 3H), 3.12 (hept., *J* = 6.9 Hz, 1 H), 4.46 (q, *J* = 6.6 Hz, 1H), 7.59 (d, *J =* 1.9 Hz, 1H), 7.70 (dd, *J =* 1.9 Hz, *J =* 0.6 Hz, 1H) ppm.
**¹³C-NMR (100 MHz, CDCl₃):** δ = 14.70 (q), 22.20 (q), 22.22 (q), 23.23 (q), 26.42 (q, q), 28.09 (d), 43.45 (s), 89.79 (d), 120.79 (d), 126.90 (d), 130.37 (s), 130.94 (s), 137.67 (s), 160.33 (s), 197.04 (s) ppm.
**MS (EI):** *m*/*z* (%) = 246 (20), 232 (8), 231 (46), 189 (11), 175 (7), 147 (10), 115 (7), 91 (8), 43 (100), 41 (9).

### 1-Methyl-4-(3-methylbut-2-enoxy)benzene

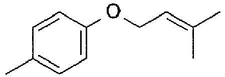

**¹H-NMR (400 MHz**, **CDCl₃):** δ = 1.70 - 1.76 (m, 3H), 1.76 - 1.82 (m, 3H), 2.25 - 2.32 (m, 3H), 4.42 - 4.53 (m, 2H), 5.44 - 5.55 (m, 1H), 6.75 - 6.86 (m, 2H), 7.01 - 7.12 (m, 2H) ppm.
**¹³C-NMR (100 MHz, CDCl₃):** δ = 18.17 (q), 20.46 (q), 25.83 (q), 64.78 (t), 114.49 (d, d), 119.91 (d), 129.77 (s), 129.83 (d, d), 137.93 (s), 156.72 (s) ppm.
**MS (EI):** *m*/*z* (%) = 109 (8), 108 (100), 107 (23), 79 (9), 77 (14), 69 (20), 53 (10), 41 (35), 39 (13), 27 (9).

### 2,3,3,5-Tetramethyl-2H-benzofuran

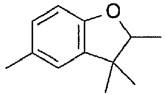

**¹H-NMR (400 MHz, CDCl₃):** δ = 1.08 (s, 3H), 1.29 (s, 3H), 1.36 (d, *J* = 6.5 Hz, 3H), 2.29 (s, 3H), 4.34 (q, *J* = 6.6 Hz, 1 H), 6.63 - 6.68 (m, 1 H), 6.86 - 6.93 (m, 2H) ppm.
**¹³C-NMR (100 MHz, CDCl₃):** ö = 14.40 (q), 20.90 (q), 23.23 (q), 25.86 (q), 43.66 (s), 88.83 (d), 109.08 (d), 123.07 (d), 128.09 (d), 129.78 (s), 137.59 (s) 156.06 (s) ppm.
**MS (EI):** *m*/*z* (%) = 176 (46), 162 (12), 161 (100), 133 (48), 115 (16), 105 (36), 91 (18), 77 (11), 41 (11), 39 (10).

### 2,3,3,5-Tetramethyl-2H-benzofuran-7-carbaldehyde

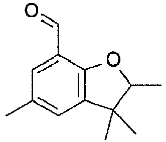

**¹H-NMR (400 MHz, CDCl₃):** δ = 1.12 (s, 3H), 1.32 (s, 3H), 1.42 (d, *J* = 6.6 Hz, 3H), 2.31 - 2.33 (m, 3H), 4.51 (q, *J* = 6.6 Hz, 1H), 7.09 - 7.11 (m, 1H), 7.35 - 7.39 (m, 1H), 10.19 - 10.20 (m, 1 H). ppm.
**¹³C-NMR (100 MHz, CDCl₃):** δ = 14.59 (q), 20.67 (q), 23.27 (q), 26.13 (q), 43.01 (s), 90.75 (d), 119.16 (s), 126.46 (d), 129.68 (d), 130.21 (s), 140.00 (s), 158.92 (s), 188.95 (d). ppm.

### 1-(2,3,3,5-Tetramethyl-2H-benzofuran-7-yl)ethanone

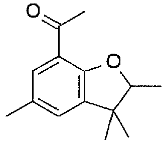

**¹H-NMR (400 MHz, CDCl₃): δ** = 1.23 (d, *J* = 7.1 Hz, 3H), 1.31 (s, 3H), 1.51 (s, 3H), 2.29 (s, 3H), 2.60 (s, 3H), 4.47 (q, *J* = 6.6 Hz, 1H), 7.04 - 7.08 (m, 1H), 7.41 - 7.54 (m, 1H) ppm.
**¹³C-NMR (100 MHz, CDCl₃):** δ = 14.69 (q), 20.59 (q), 22.09 (q), 28.08 (q), 31.23 (q), 45.04 (s), 91.05 (d), 120.21 (s), 127.54 (d), 129.31 (s), 129.75 (d), 135.33 (s), 156.81 (s), 197.55 (s) ppm.

### 2,2,3,5-Tetramethyl-3H-benzofuran

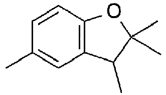

**¹H-NMR (4.00 MHz, CDCl₃):** ö = 1.21 (d, *J* = 7.1 Hz, 3H), 1.26 - 1.27 (m, 3H), 1.44 - 1.47 (m, 3H), 2.28 (s, 3H), 3.11 (q, *J* = 7.1 Hz, 1H), 6.61 (d, *J* = 8.5 Hz, 1H), 6.88 - 6.92 (m, 2H) ppm.

### 1-Methyl-2-(3-methylbut-2-enoxy)benzene

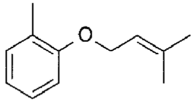

**¹H-NMR (400 MHz, CDCl₃**): δ = 1.73 (s, 3H), 1.79 (s, 3H), 2.23 (s, 3H), 4.52 (d, *J* = 6.5 Hz, 2H), 5.58 - 5.43 (m, 1H), 6.89 - 6.78 (m, 2H), 7.18 - 7.07 (m, 2H) ppm.
¹³**C-NMR (100 MHz, CDCl₃):** δ = 16.32 (q), 18.23 (q), 25.79 (q), 65.00 (t), 111.36 (d), 120.20 (d), 120.33 (d), 126.63 (d), 127.02 (s), 130.62 (d), 137.08 (s), 157.05 (s) ppm.
**MS (EI):** *m*/*z* (%) = 109 (8), 108 (100), 107 (14), 80 (4), 79 (5), 77 (8), 69 (26), 41 (27), 39 (6), 27 (4).

### 2,3,3,7-Tetramethyl-2H-benzofuran

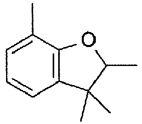

**¹H-NMR (400 MHz, CDCl₃):** δ = 1.08 (s, 3H), 1.30 (s, 3H), 1.38 (d, *J* = 6.6 Hz, 3H), 2.22 (s, 3H), 4.35 (q, *J* = 6.6 Hz, 1 H), 6.78 (dd, *J* = 7.4 Hz, 1 H), 6.89 - 6.95 (m, 2H) ppm.
**¹³C-NMR (100 MHz, CDCl₃):** δ = 14.48 (q), 15.15 (q), 23.27 (q), 26.03 (q), 43.81 (s), 88.46 (d), 119.62 (s), 119.76 (d), 120.37 (d), 129.02 (d), 136.88 (s), 156.58 (s) ppm.
**MS (EI):** *m*/*z* (%) = 176 (49), 161 (100), 133 (45), 117 (14), 115 (18), 105 (30), 91 (28), 77 (17), 41 (14), 39 (17).

### 1-(2,2,3,7-Tetramethyl-3H-benzofuran-5-yl)ethanone

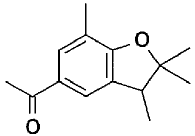

**¹H-NMR (400 MHz, CDCl₃**): δ = 1.25 (d, *J* = 7.2 Hz, 3H), 1.29 (s, 3H), 1.50 (s, 3H), 2.22 (s, 3H), 2.53 (s, 3H), 3.17 (q, *J* = 7.0 Hz, 1H), 7.59 - 7.60 (m, 1H), 7.61 - 7.63 (m, 1H) ppm.
**¹³C-NMR (100 MHz, CDCl₃**): δ = 14.74 (q), 15.18 (q), 22.09 (q), 26.43 (q), 28.15 (q), 45.47 (d), 91.08 (s), 119.29 (s), 122.30 (d), 130.29 (s), 131.39 (d), 132.69 (s), 161.07 (s), 197.00 (s) ppm.
**MS (EI):** *m*/*z* (%) = 219 (7), 218 (43), 204 (14), 203 (100), 161 (15), 147 (12), 115 (11), 91 (11), 43 (66), 41 (7).

### 1-Methyl-3-(3-methylbut-2-enoxy)benzene

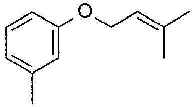

**¹H-NMR (400 MHz, CDCl₃):** δ = 1.74 (s, 3H), 1.79 (s, 3H), 2.32 (s, 3H), 4.49 (d, *J* = 6.8 Hz, 2H), 5.42 - 5.54 (m, 1H), 6.67 - 6.80 (m, 3H), 7.15 (ddd, *J* = 8.0 Hz, *J* = 7.2 Hz, *J* = 0.9 Hz, 1H) ppm.
**¹³C-NMR (100 MHz, CDCl**₃): δ = 18.17 (q), 21.55 (q), 25.83 (q), 64.58 (t), 111.50 (d), 115.48 (d), 119.83 (d), 121.40 (d), 129.12 (d), 137.97 (s), 139.40 (s), 158.87 (s) ppm.
**MS (EI):** *m*/*z* (%) = 109 (8), 108 (100), 107 (18), 79 (8), 77 (12), 69 (24), 53 (8), 41 (36), 39 (12), 27 (7).

### 2,3,3,6-tetramethyl-2H-benzofuran

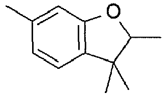

**¹H-NMR (400 MHz, CDCl₃):** δ = 1.08 (s, 3H), 1.29 (s, 3H), 1.35 (d, *J* = 6.6 Hz, 3H), 2.30 (s, 3H), 4.35 (q, *J* = 6.6 Hz, 1H), 6.59 (dd, *J* = 1.4 Hz, *J* = 0.7 Hz, 1H), 6.69 (ddd, *J* = 7.5 Hz, *J =* 1.5 Hz, *J =* 0.7 Hz, 1H), 6.96 (dd, *J =* 7.4 Hz, *J =* 0.4 Hz, 1H) ppm.
**¹³C-NMR (100 MHz, CDCl₃):** δ = 14.48 (q), 21.49 (q), 23.30 (q), 26.11 (q), 43.38 (s), 88.99 (d), 110.30 (d), 121.19 (d), 122.06 (d), 134.73 (s), 137.89 (s), 158.42 (s) ppm.

### 1-(2,3,3,4-Tetramethyl-2H-benzofuran-5-yl)ethanone

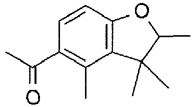

**¹H-NMR (400 MHz, CDCl₃**): δ = 1.16 (s, 3H), 1.37 (d, *J* = 6.5 Hz, 3H), 1.42 (s, 3H), 2.53 (s, 3H), 2.53 (s, 3H), 4.30 (q, *J* = 6.5 Hz, 1H), 6.63 (dd, *J* = 8.4 Hz, *J* = 0.5 Hz, 1H), 7.56 (dd, *J* = 8.4 Hz, *J* = 0.4 Hz, 1 H) ppm.
**¹³C-NMR (100 MHz, CDCl₃**): δ = 13.67 (q), 16.27 (q), 20.86 (q), 25.25 (q), 29.73 (q), 44.55 (s), 88.82 (d), 106.81 (d), 131.30 (d), 132.12 (s), 136.01 (s), 136.68 (s), 161.18 (s), 200.72 (s) ppm.

### 1-(2,3,3,4-Tetramethyl-2H-benzofuran-7-yl)ethanone

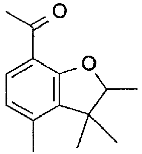

**¹H-NMR (400 MHz, CDCl₃**): δ = 1.16 (s, 3H), 1.38 - 1.44 (m, 6H), 2.39 (s, 3H), 2.60 (s, 3H), 4.38 (q, *J* = 6.5 Hz, 1 H), 6.66 (dd, *J* = 8.1 Hz, *J* = 0.6 Hz, 1 H), 7.59 (dd, *J* = 8.1 Hz, *J* = 0.4 Hz, 1H) ppm.
**¹³C-NMR (100 MHz, CDCl₃**): δ = 13.94 (q), 18.46 (q), 20.79 (q), 25.07 (q), 31.05 (q), 43.81 (s), 88.82 (d), 119.20 (s), 123.51 (d), 127.91 (d), 135.77 (s), 140.11 (s), 159.14 (s), 197.11 (s) ppm.

### 1-(2,3,3,6-Tetramethyl-2H-benzofuran-5-yl)ethanone

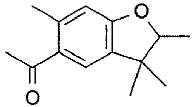

**¹H-NMR (400 MHz, CDCl₃):** δ = 1.12 (s, 3H), 1.34 (s, 3H), 1.38 (d, *J* = 6.6 Hz, 3H), 2.53 (s, 3H), 2.56 (s, 3H), 4.45 (q, *J* = 6.6 Hz, 1 H), 6.58 - 6.65 (m, 1 H), 7.48 (s, 1 H) ppm.
**¹³C-NMR (100 MHz, CDCl₃):** δ = 14.59 (q), 22.76 (q), 23.35 (q), 26.33 (q), 29.37 (q), 43.30 (s), 90.05 (d), 113.03 (d), 124.86 (d), 130.47 (s), 134.99 (s), 141.40 (s), 161.13 (s), 199.53 (s) ppm.

### 1-Isopropyl-4-methyl-2-(3-methylbut-2-enoxy)benzene

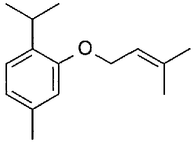

**¹H-NMR (400 MHz, CDCl₃**): δ = 1.19 (d, *J* = 6.9 Hz, 6H), 1.73 (s, 3H), 1.78 (s, 3H), 2.31 (s, 3H), 3.30 (hept., *J* = 6.9 Hz, 1 H), 4.50 (dt, *J* = 6.6 Hz, *J* = 1.0 Hz, 2H), 5.43 - 5.55 (m, 1H), 6.65 - 6.68 (m, 1H), 6.73 (dd, *J* = 7.7 Hz, *J* = 1.8 Hz, 1H), 7.08 (d, *J* = 7.7 Hz, 1H) ppm.
**¹³C-NMR (100 MHz, CDCl₃):** δ = 18.22 (q), 21.36 (q), 22.84 (q, q), 25.77 (q), 26.47 (d), 65.08 (t), 112.74 (d), 120.46 (d), 121.06 (d), 125.82 (d), 134.38 (s), 136.11 (s), 136.89 (s), 155.98 (s) ppm.
**MS (EI):** *m*/*z* (%) = 150 (42), 136 (11), 135 (100), 115 (6), 91 (13), 77 (6), 69 (30), 41 (35), 39 (8), 27 (5).

### 7-Isopropyl-2,3,3,4-tetramethyl-2H-benzofuran

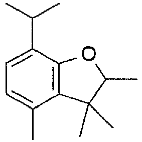

**H-NMR (400 MHz, CDCl₃):** δ = 1.12 (s, 3H), 1.22 (dd, *J* = 6.9 Hz, 6H), 1.36 (d, *J* = 6.5 Hz, 3H), 1.38 (s, 3H), 2.33 (s, 3H), 3.09 (hept., *J =* 6.3 Hz, 1H), 4.25 (q, *J =* 6.5 Hz, 1H), 6.60 (d, *J* = 7.7 Hz, 1 H), 6.91 (d, *J* = 7.7 Hz, 1 H) ppm.
**¹³C-NMR (100** MHz, **CDCl₃):** δ = 13.88 (q), 17.94 (q), 20.86 (q), 22.51 (q), 22.62 (q), 25.10 (q), 27.48 (d), 44.61 (s), 87.49 (d), 122.94 (d), 124.37 (d), 128.40 (s), 131.46 (s), 133.64 (s), 155.92 (s) ppm.
**MS (EI):** *m*/*z* (%) = 218 (46), 203 (93), 161 (100), 147 (26), 133 (26), 115 (15), 105 (16), 91 (16), 43 (23), 41 (22).

### 1-(7-Isopropyl-2,3,3,4-tetramethyl-2H-benzofuran-5-yl)ethanone

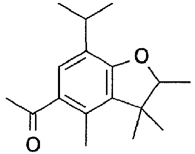

**¹H-NMR (400 MHz, CDCl₃**): δ = 1.15 (s, 3H), 1.24 (d, *J* = 6.9 Hz, 6H), 1.37 (d, *J* = 6.5 Hz, 3H), 1.41 (s, 3H), 2.49 (s, 3H), 2.54 (s, 3H), 3.09 (hept., *J* = 6.7 Hz, 1H), 4.28 (q, *J* = 6.5 Hz, 1 H), 7.42 (s, 1 H) ppm.
**¹³C-NMR (100 MHz, CDCl₃)**: δ = 13.80 (q), 15.97 (q), 20.87 (q), 22.25 (q), 22.34 (q), 25.36 (q), 27.69 (d), 29.84 (q), 44.66 (s), 88.41 (d), 127.50 (s), 127.80 (d), 132.15 (s), 133.57 (s), 135.50 (s), 158.83 (s), 201.18 (s) ppm.
**MS (EI):** *m*/*z* (%) = 260 (36), 246 (18), 245 (100), 203 (13), 161 (16), 128 (7), 115 (10), 91 (7), 43 (91), 41 (10).

### 7-Isopropyl-2,3,3,4-tetramethyl-2H-benzofuran-5-carbaldehyde oxime

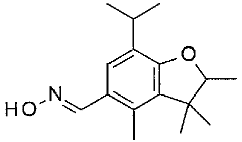

**¹H-NMR (400 MHz, CDCl₃**): δ = 1.14 (s, 3H), 1.20 - 1.24 (m, 6H), 1.37 (d, *J* = 6.5 Hz, 3H), 1.40 (s, 3H), 2.38 (s, 3H), 3.08 (hept., *J* = 6.9 Hz, 1H), 4.27 (q, *J* = 6.5 Hz, 1 H), 7.36 - 7.40 (m, 1H), 8.24 (s, 1H), 8.38 - 8.42 (m, 1H) ppm.
**¹³C-NMR (100 MHz, CDCl₃**): δ = 13.81 (q), 14.20 (q), 21.12 (q), 22.31 (q), 22.41 (q), 25.49 (q), 27.61 (d), 44.67 (s), 88.07 (d), 123.50 (s), 124.25 (d), 128.95 (s), 131.11 (s), 134.49 (s), 149.66 (d), 157.75 (s) ppm.
**MS (EI):** *m*/*z* (%) = 261 (90), 246 (100), 244 (28), 243 (30), 228 (78), 204 (49), 187 (33), 186 (90), 43 (30), 41 (23).

### 7-Isopropyl-2,3,3,4-tetramethyl-2H-benzofuran-5-carbonitrile

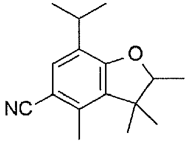

**¹H-NMR (400 MHz, CDCl₃**): ö = 1.14 (s, 3H), 1.20 (d, *J* = 6.9 Hz, 6H), 1.37 (d, *J* = 6.5 Hz, 3H), 1.39 (s, 3H), 2.50 (s, 3H), 3.06 (hept., *J* = 6.9 Hz, 1 H), 4.33 (q, *J* = 6.6 Hz, 1 H), 7.27 - 7.29 (m, 1H) ppm.
**¹³C-NMR (100 MHz, CDCl₃**): δ = 13.85 (q), 16.01 (q), 20.93 (q), 22.05 (q), 22.12 (q), 25.15 (q), 27.44 (d), 44.66 (s), 88.65 (d), 105.26 (s), 119.55 (s), 129.68 (s), 130.37 (d), 134.83 (s), 135.78 (s), 159.64 (s) ppm.
**MS** (EI): *m*/*z* (%) = 244 (6), 243 (33), 229 (11), 228 (67), 187 (14), 186 (100), 172 (19), 115 (5), 106 (5), 43 (5).

### 3-Methylbut-2-enoxybenzene

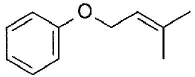

**¹H-NMR (200 MHz, CDCl₃):** δ = 1.75 (s, 3H), 1.80 (s, 3H), 4.51 (d, *J* = 6.8 Hz, 2H), 5.40 - 5.60 (m, 1H), 6.85 - 7.01 (m, 3H), 7.14 - 7.39 (m, 2H) ppm.
**¹³C-NMR (50 MHz, CDCl₃):** δ = 18.12 (q), 25.78 (q), 64.50 (t), 114.48 (d, d), 119.67 (d), 120.43 (d), 129.25 (d, d), 137.81 (s), 158.65 (s) ppm.
**MS (EI):** *m*/*z* (%) = 162 (7), 95 (7), 94 (100), 69 (32), 67 (8), 66 (5), 65 (8), 53 (5), 41 (33), 39 (12).

### 2,3,3-Trimethyl-2H-benzofuran

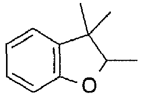

**¹H-NMR (400 MHz, CDCl₃**): δ = 1.09 (s, 3H), 1.31 (s, 3H), 1.37 (d, *J* = 6.6 Hz, 3H), 4.37 (q, *J* = 6.5 Hz, 1 H), 6.74 - 6.79 (m, 1 H), 6.83 - 6.90 (m, 1 H), 7.04 - 7.14 (m, 2H) ppm.
**¹³C-NMR (100 MHz, CDCl₃):** δ = 14.43 (q), 23.26 (q), 25.96 (q), 43.61 (s), 88.76 (d), 109.53 (d), 120.53 (d), 122.46 (d), 127.76 (d), 137.57 (s), 158.18 (s) ppm.
**MS (EI)**: *m*/*z* (%) = 162 (50), 148 (12), 147 (100), 131 (8), 119 (41), 103 (8), 91 (34), 77 (10), 41 (7), 39 (7).

### 1-(2,3,3,4,7-Pentamethyl-2H-benzofuran-5-yl)ethanone

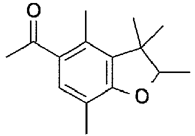

**¹H-NMR (400 MHz, CDCl₃):** δ = 1.15 (s, 3H), 1.38 (d, *J =* 6.5 Hz, 3H), 1.41 (s, 3H), 2.20 (s, 3H), 2.49 (s, 3H), 2.53 (s, 3H), 4.28 (q, *J* = 6.5 Hz, 1 H), 7.36 - 7.38 (m, 1 H) ppm.
**¹³C-NMR (100 MHz, CDCl₃):** δ = 13.72 (q), 14.96 (q), 15.95 (q), 20.85 (q), 25.31 (q), 29.81 (q), 44.79 (s), 88.52 (d), 116.57 (s), 131.90 (s), 132.23 (d), 133.79 (s), 135.27 (s), 159.67 (s), 200.96 (s) ppm.

### 2,3,3,4,7-Pentamethyl-2H-benzofuran-5-carbaldehyde

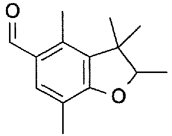

**¹H-NMR (400 MHz, CDCl₃):** δ = 1.17 (s, 3H), 1.40 (d, *J* = 6.6 Hz, 3H), 1.43 (s, 3H), 2.21 (s, 3H), 2.64 (s, 3H), 4.35 (q, *J* = 6.6 Hz, 1H), 7.44 - 7.45 (m, 1H), 10.06 (s, 1H) ppm.
**¹³C-NMR (100 MHz, CDCl₃**): δ = 13.78 (q), 13.82 (q), 14.73 (q), 21.11 (q), 25.47 (q), 44.45 (s), 89.01 (d), 117.93 (s),128.58 (s), 135.16 (s), 135.81 (s), 135.96 (d), 161.90 (s), 191.57 (d) ppm.

### 2,3,3,4,6,7-Hexamethyl-2H-benzofuran-5-carbaldehyde

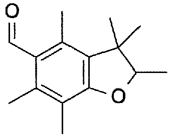

**¹H-NMR (400 MHz, CDCl₃**): δ = 1.15 (s, 3H), 1.38 (d, *J* = 6.5 Hz, 3H), 1.41 (s, 3H), 2.14 (s, 3H), 2.47 (s, 3H), 2.56 (s, 3H), 4.28 (q, *J* = 6.5 Hz, 1H), 10.51 (s, 1H) ppm.
**¹³C-NMR (100 MHz, CDCl₃**): δ = 11.54 (q), 13.76 (q), 15.12 (q), 15.46 (q), 21.14 (q), 25.62 (q), 44.70 (s), 88.38 (d), 117.46 (s), 127.48 (s), 132.36 (s), 135.74 (s), 141.84 (s), 160.93 (s), 193.16 (d) ppm.

### 1-(2,3,3,4,6,7-Hexamethyl-2H-benzofuran-5-yl)ethanone

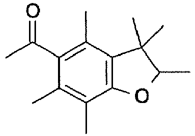

**¹H-NMR (400 MHz, CDCl₃):** δ = 1.10 (s, 3H), 1.36 (d, *J* = 6.4 Hz, 3H), 1.37 (s, 3H), 2.09 (s, 6H), 2.20 (s, 3H), 2.45 (s, 3H), 4.23 (q, *J* = 6.5 Hz, 1 H) ppm.
**¹³C-NMR (100 MHz, CDCl₃**): δ = 11.45 (q), 13.73 (q), 14.79 (q), 16.15 (q), 20.90 (q), 25.25 (q), 33.23 (q), 45.00 (s), 87.81 (d), 116.68 (s), 125.67 (s), 130.96 (s), 131.60 (s), 136.62 (s), 157.06 (s), 209.88 (s). ppm.

### 1-(6-Isopropyl-2,3,3,4-tetramethyl-2H-benzofuran-5-yl)ethanone

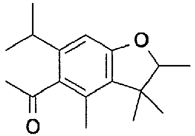

**¹H-NMR (400 MHz, CDCl₃**): δ = 6.58 (s, 1H), 4.26 (q, *J* = 6.5 Hz, 1H), 2.71 (hept, *J* = 6.8 Hz, 1 H), 2.47 (s, 3H), 2.22 (d, *J* = 0.4 Hz, 3H), 1.38 (s, 3H), 1.35 (d, *J* = 6.5 Hz, 3H), 1.20 (d, *J* = 6.8 Hz, 3H), 1.19 (d, *J* = 6.8 Hz, 3H), 1.12 (s, 3H) ppm.
**¹³C-NMR (100 MHz, CDCl₃**): δ = 209.68 (s), 159.03 (s), 144.48 (s) , 135.52 (s), 132.54 (s), 128.70 (s), 104.60 (d), 88.37 (d), 44.63 (s), 33.79 (q), 31.05 (d), 25.24 (q), 24.42 (q), 24.25 (q), 21.05 (q), 15.10 (q), 13.80 (q) ppm.

### 1-(6-Isopropyl-2,3,3,4-tetramethyl-2H-benzofuran-7-yl)ethanone

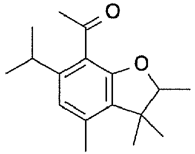

**¹H-NMR (400 MHz, CDCl₃**): δ = 6.59 (s, 1H), 4.31 (q, *J* = 6.5 Hz, 1H), 3.16 (hept, *J* = 6.9 Hz, 1H), 2.55 (s, 3H), 2.34 (d, *J* = 0.6 Hz, 4H), 1.37 (s, 3H), 1.34 (d, *J* = 6.5 Hz, 3H), 1.19 (d, *J* = 6.8 Hz, 3H), 1.18 (d, *J* = 6.8 Hz, 3H), 1.12 (s, 3H) ppm.
**¹³C-NMR (100 MHz, CDCl₃**): δ = 203.40 (s), 156.30 (s), 146.47 (s), 136.20 (s), 132.01 (s), 121.39 (s), 120.61 (d), 88.67 (d), 43.89 (s), 32.54 (q), 29.13 (d), 25.12 (q), 24.36 (q), 24.24 (q), 20.86 (q), 18.39 (q), 13.91 (q) ppm.

### Anwendungsbeispiele:

### Beispiel 6: Parfümkomposition (Riechstoffmischung / Riechstoffkomposition):

| | |
|---|---|
| Agrumex LC | 10.00 |
| Amarocit® 10%ig in DPG | 10.00 |
| Ambroxid krist. | 10.00 |
| Basilikumöl | 10.00 |
| Calone 1951 10%ig in DPG | 10.00 |
| Cedernholzöl | 10.00 |
| Cedrol Krist | 50.00 |
| Citral 10%ig in DPG | 10.00 |
| Citonellol | 5.00 |
| Cumarin | 10.00 |
| Cyclogalbanat® 10%ig in DPG | 15.00 |
| Dihydromyrcenol | 80.00 |
| Farenal® 10%ig in DPG | 5.00 |
| Galbex 10%ig in DPG | 25.00 |
| Geraniol | 80.00 |
| Geranyl nitrile | 40.00 |
| Hedion | 90.00 |
| Helional | 20.00 |
| Heliotropin | 5.00 |
| Hexenol cis-3 10%ig in DPG | 15.00 |
| Hexenylsalicylat cis-3 | 10.00 |
| Beta-Ionon | 5.00 |
| Iso E Super | 180.00 |
| Isodamascon® 10%ig in DPG | 10.00 |
| Isogalbanat | 20.00 |
| Isoraldein 70 | 20.00 |
| Ketamber 10%ig in TEC | 25.00 |
| Lavandinoel Grosso Nat. | 15.00 |
| Lilial | 20.00 |
| Linalool | 20.00 |
| Linalylacetat | 40.00 |
| Mandarinenoel brasil. Grün | 50.00 |
| Timberol® | 40.00 |
| Vanillin | 5.00 |
| Veloutone 10%ig in DPG | 20.00 |
| Ysamber K® | 10.00 |
| Gesamt | 1000.00 |

| | |
|---|---|
| DPG: Dipropylenglycol, TEC = Triethylcitrat Geruchsbeschreibung der Parfümkomposition ohne Zusatz: frisch, holzig. | |

Nach Meinung der Parfümeure wird diese Parfümkomposition zum Beispiel durch den Zusatz von 3 Gew.-% an 1-(7-Isopropyl-2,3,3,4-tetramethyl-2H-benzofuran-5-yl)-ethanone (Verbindung 2) frischer, strahlender, abgerundeter und harmonischer, wobei eine moschusartige und süße Note hinzukommt und die holzigen und blumigen Aspekte verstärkt werden. Die erfindungsgemäß zu verwendenden Verbindungen der Formel (I) und entsprechende Mischungen davon verleihen einer vorstehenden (oder einer anderen) Komposition durch ihren Eigengeruch und/oder durch ihre modifizierende und/oder verstärkende Wirkung (Booster-Wirkung) vorteilhafterweise einen eigenen Charakter und verbinden die unterschiedlichen geruchlichen Elemente.

### Beispiel 7: Parfümkomposition (Riechstoffkomposition)

| | |
|---|---|
| Allylcyclohexylpropionat | 3.00 |
| Amylsalicylat | 2.00 |
| Benzylacetat | 64.00 |
| Citronellol | 122.00 |
| Citral 10% ig in DPG | 2.00 |
| Cyclamenaldehyd | 10.00 |
| Dihydromyrcenol | 3.00 |
| Dimethylbenzylcarbinylacetat | 3.00 |
| Ethylsalicylat 10% ig in DPG | 2.00 |
| Eugenol | 3.00 |
| Indoflor 10%ig in DPG | 16.00 |
| Galbaniff | 164.00 |
| Geraniol | 35.00 |
| Dihydromethyljasmonate | 6.00 |
| Heliotropin | 4.00 |
| Hexylzimtaldehyd | 121.00 |
| Vertocitral | 4.00 |
| Hydroxycitronellal | 42.00 |
| Indol | 2.00 |
| Isobutylsalicylat | 6.00 |
| Lavandinoel Grosso Nat. | 6.00 |
| Lactoscatone | 10.00 |
| Lilial | 190.00 |
| Linalool | 35.00 |
| Linalylacetat | 10.00 |
| Methylanthranilat 10%ig in DPG | 5.00 |
| Nerol | 10.00 |
| Orangenoel | 6.00 |
| Paraxonal | 4.00 |
| Phenylacetaldehyddimethylacetal | 6.00 |
| Phenylethylalkohol | 75.00 |
| Rosatol 10%ig in DPG | 6.00 |
| Sandelholzoel | 3.00 |
| Sandranol | 16.00 |
| TCD-Alcohol M | 2.00 |
| Trifernal | 2.00 |
| Gesamt | 1000.00 |

| | |
|---|---|
| DPG: Dipropylenglycol Geruchsbeschreibung der Parfümkomposition ohne Zusatz: blumig, Maiglöckchen. | |

Nach Meinung der Parfümeure erwacht diese Parfümkomposition insbesondere durch den Zusatz von 6 Gew.-% von 1-(7-*tert*.-Butyl-2,3,3-trimethyl-2H-benzofuran-5-yl)ethanone (Verbindung 2) zu neuem Leben. Der Eindruck von Blumigkeit wird erheblich verstärkt. Die Komposition wirkt strahlender, abgerundeter und harmonischer, wobei eine moschusartige und natürliche Note hinzukommt. Die erfindungsgemäß zu verwendenden Verbindungen der Formel (I) und entsprechende Mischungen davon verleihen der Komposition durch ihren Eigengeruch sowie durch ihre modifizierende und verstärkende Wirkung (Booster-Wirkung) einen eigenen Charakter und verbinden die unterschiedlichen geruchlichen Elemente.

### Beispiel 8: Shampoo

Eine Mischung von 1-(7-*tert*-Butyl-2,3,3-trimethyl-2H-benzo-furan-5-yl)ethanone (Verbindung 2) und 7-Isopropyl-2,3,3,4-tetramethyl-2H-benzofuran-5-carbonitrile wurde in einer Dosierung von 0,5 Gew.-% in eine Shampoo-Grundmasse folgender Zusammensetzung eingearbeitet:

| | |
|---|---|
| Natriumlaurylethersulfat | 12% |
| (z.B. Texapon NSO, Fa. Cognis Deutschland GmbH) | |
| Cocamidopropylbetain | 2% |
| (z.B. Dehyton K, Fa. Cognis Deutschland GmbH) | |
| Natriumchlorid | 1,4% |
| Citronensäure | 1,3% |
| Phenoxyethanol, Methyl-, Ethyl-, Butyl-, und Propylparaben | 0,5% |
| Wasser | 82,8% |

Der pH-Wert der Shampoo-Grundmasse lag bei etwa 6. Hieraus wurden 100 mL einer 20 Gew.%-igen wässrigen Shampoo-Lösung hergestellt. In dieser Shampoo-Lösung wurden 2 Haarsträhnchen gemeinsam für 2 Minuten gewaschen und anschließend 20 Sekunden unter fließendem handwarmen Wasser gespült. Eine Haarsträhne wurde nass in Aluminiumfolie eingepackt und die zweite Haarsträhne mit einem Fön getrocknet. Beide Haarsträhnen wurden von einem Panel geruchlich beurteilt.
Geruchsbeschreibung jeweils: sehr stark strahlend, moschusartig, exaltierend, weich holzig, erinnert an Moschuskörner.

### Beispiel 9: Weichspüler

Die Parfümkomposition aus Beispiel 6 (nach Zusatz von 6 Gew.-% an Verbindung der Formel (I) wie beschrieben) wurde in einer Dosierung von 0,5 Gew.-% in eine Weichspüler-Grundmasse folgender Zusammensetzung eingearbeitet:

| | |
|---|---|
| Quarternäres Ammoniummethosulfat (Esterquat), ca. 90% | 5,5% |
| (z.B. Rewoquat WE 18, Fa. Witco Surfactants GmbH) | |
| Alkyldimethylbenzylammoniumchlorid, ca. 50% | 0,2% |
| (z.B. Preventol R50, Fa. Bayer AG) | |
| Farblösung, ca. 1%-ig | 0,3% |
| Wasser | 94,0% |

Der pH-Wert der Weichspüler-Grundmasse lag im Bereich von 2 bis 3. Zwei Stofflappen wurden mit 370 g einer 1%-igen wässrigen Weichspüler-Lösung auf Basis der 0,5% Gew.-% Verbindung der Formel (I) umfassenden Weichspüler-Grundmasse in einer Linetest-Maschine im Weichspülprogramm 30 Minuten bei 20°C gespült. Die Lappen wurden ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt.

Geruchsbeschreibung jeweils: blumig, holzig, frisch, strahlend, moschusartig und holzige Aspekte mit leichten süßen Untertönen, abgerundeter und harmonischer Geruchseindruck.

### Beispiel 10: Waschpulver

Die Parfümölkomposition aus Beispiel 7 (nach Zusatz von 6 Gew.-% an Verbindung der Formel (I) wie beschrieben) wurde in einer Dosierung von 0,4 Gew.-% in eine Waschpulver-Grundmasse der folgenden Rezeptur eingearbeitet:

| | |
|---|---|
| Lineares Na-Alkylbenzolsulfonat | 8,8 % |
| Ethoxylierter Fettalkohol C12-18 (7 EO) | 4,7 % |
| Na-Seife | 3,2 % |
| Entschäumer | |
| DOW CORNING(R) 2-4248S | |
| POWDERED ANTIFOAM, | |
| Silikonöl auf Zeolith als Trägermaterial | 3,9 % |
| Zeolith 4A | 28,3 % |
| Na-Carbonat | 11,6 % |
| Na-Salz eines Copolymers aus Acryl- und Maleinsäure (Sokalan CP5) | 2,4 % |
| Na-Silikat | 3,0 % |
| Carboxymethylcellulose | 1,2 % |
| Dequest 2066 | 2,8 % |
| ([[(Phosphonomethyl)imino]bis[(ethylennitrilo)bis (methylen)]]tetrakis-phosphonsäure, Natriumsalz) Optischer Aufheller | 0,2 % |
| Na-Sulfat | 6,5 % |
| Protease | 0,4 % |
| Natriumperborattetrahydrat | 22,0 % |
| TAED | 1,0 % |

Zwei Stofflappen wurden mit 370 g einer 1%-igen wässrigen Waschpulverlauge auf Basis des 0,4 Gew.-% der Parfümölkomposition aus Beispiel 7 umfassenden Waschpulver-Grundmasse (der pH-Wert der Waschpulverlauge liegt deutlich im basischen Bereich) in einer Linetest-Maschine im Hauptwaschgang 45 Minuten bei 60°C gewaschen. Die Lappen wurden zunächst 5 Minuten mit kaltem Wasser gespült, ausgewrungen und anschließend 20 Sekunden geschleudert. Ein Lappen wurde nass eingeschweißt, und einer zum Trocknen aufgehängt. Anschließend wurden beide Lappen durch ein Panel geruchlich beurteilt.

Geruchsbeschreibung jeweils: stark blumig, strahlend, moschusartig und natürliche Note mit leichten süßen und holzigen Untertönen, abgerundeter und harmonischer Geruchseindruck.

## Patentansprüche

1. Verwendung
- einer Verbindung der Formel (I) 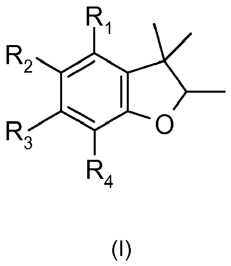 oder
- einer Mischung aus zwei, drei, vier, fünf, sechs oder mehreren verschiedenen Verbindungen der Formel (I),
wobei für die Reste der Verbindung der Formel (I) bzw. die Reste sämtlicher Verbindungen der Formel (I) jeweils gilt, dass R₁, R₂, R₃ und R₄ jeweils unabhängig voneinander Wasserstoff, Methyl, Ethyl, Propyl, Isopropyl, Butyl, sec-Butyl, tert-Butyl, iso-Butyl, Acetyl, Formyl oder Cyano bedeuten,
als Riech- und/oder Aromastoff bzw. als Riechstoff- und/oder Aromastoffmischung.

2. Verwendung einer Verbindung oder einer Mischung nach Anspruch 1 als Riech- und/oder Aromastoff mit moschusartiger Note bzw. als Riechstoff- und/oder Aromastoffmischung mit moschusartiger Note.

3. Verwendung einer Verbindung oder einer Mischung nach einem der Ansprüche 1 oder 2 zum Vermitteln, Modifizieren und/oder Verstärken eines Geruchs und/oder eines Geschmacks, vorzugsweise eines moschusartigen Geruchs, insbesondere eines strahlend-moschusartigen Geruchs, eines exaltierenden Geruchs und/oder eines holzigen, insbesondere eines weich holzigen Geruchs.

4. Verwendung nach einem der vorangehenden Ansprüche, wobei für die Verbindung der Formel (I) bzw. eine, mehrere oder sämtliche Verbindungen der Formel (I) jeweils unabhängig voneinander gilt:
R₁ bedeutet Wasserstoff, Methyl, Acetyl oder Formyl, vorzugsweise Wasserstoff oder Methyl,
und/oder
R₂ bedeutet Methyl, Formyl oder Acetyl, vorzugsweise Formyl oder Acetyl,
und/oder
R₃ bedeutet Wasserstoff oder Methyl
und/oder
R₄ bedeutet Methyl, Ethyl, Propyl, Isopropyl, Butyl, sec-Butyl, tert-Butyl oder iso-Butyl, vorzugsweise Methyl, Ethyl, Isopropyl oder tert-Butyl.

5. Verwendung nach einem der vorangehenden Ansprüche, wobei für die Verbindung der Formel (I) bzw. eine, mehrere oder sämtliche Verbindungen der Formel (I) jeweils unabhängig voneinander gilt:
R₁ bedeutet Wasserstoff, Methyl, Acetyl oder Formyl, vorzugsweise Wasserstoff oder Methyl,
R₂ bedeutet Methyl, Formyl oder Acetyl, vorzugsweise Formyl oder Acetyl,
R₃ bedeutet Wasserstoff oder Methyl und
R₄ bedeutet Methyl, Ethyl, Propyl, Isopropyl, Butyl, sec-Butyl, tert-Butyl oder iso-Butyl, vorzugsweise Methyl, Ethyl, Isopropyl oder tert-Butyl.

6. Verwendung nach einem der vorangehenden Ansprüche, wobei die Verbindung der Formel (I) bzw. eine, mehrere oder sämtliche Verbindungen der Formel (I) ausgewählt ist bzw. jeweils unabhängig voneinander ausgewählt sind aus der Gruppe bestehend aus den folgenden Verbindungen 1 bis 8:

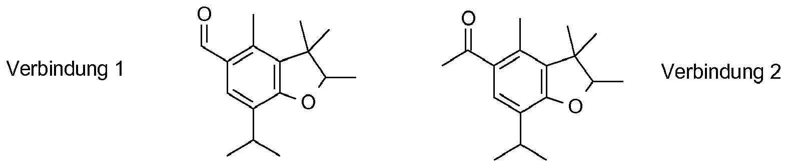

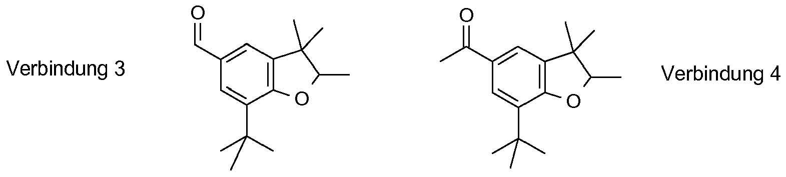

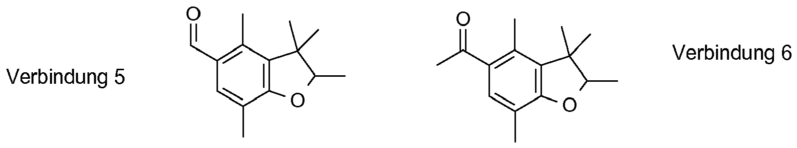

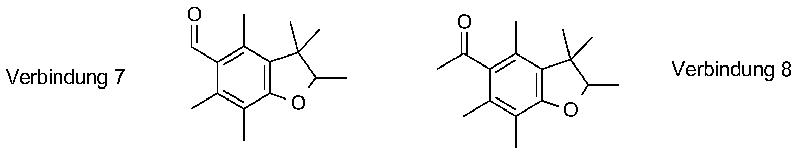

7. Riechstoff- und/oder Aromastoffmischung, umfassend oder bestehend aus
(a) einer Verbindung der Formel (I) oder einer Mischung aus zwei, drei, vier, fünf, sechs oder mehreren verschiedenen Verbindungen der Formel (I), wie jeweils in einem der vorangehenden Ansprüche definiert, vorzugsweise wie jeweils in einem der Ansprüche 4 bis 6 definiert, insbesondere wie in Anspruch 5 definiert,
und
(b) einem, zwei, drei oder mehreren weiteren Riech- und/oder Aromastoffen, wobei der bzw. die weiteren Riech- bzw. Aromastoffe keine Verbindungen der Formel (I) sind.

8. Riechstoff- und/oder Aromastoffmischung nach Anspruch 7, wobei der bzw. ein, zwei, drei, mehr oder sämtliche der weiteren Riech- bzw. Aromastoffe gemäß Bestandteil (b) einen holzigen, einen moschusartigen und/oder einen blumigen Geruch und/oder Geschmack vermitteln.

9. Riechstoff- und/oder Aromastoffmischung nach einem der Ansprüche 7 oder 8, wobei
die Gesamtmenge an Verbindungen der Formel (I) im Bereich von 0,00001 bis 99,9 Gew.-%, vorzugsweise im Bereich von 0,001 bis 70 Gew.-%, besonders bevorzugt im Bereich von 0,01 bis 50 Gew.-%, liegt, bezogen auf das Gesamtgewicht der Riechstoff- bzw. Aromastoffmischung,
und/oder
das Gewichtsverhältnis der Gesamtmenge an Bestandteil (a) zur Gesamtmenge an Bestandteil (b) in der Riechstoff- bzw. Aromastoffmischung im Bereich von 1 : 100.000 bis 10 : 1 liegt, vorzugsweise im Bereich von 1 : 10.000 bis 5 : 1, insbesondere im Bereich von 1 : 1.000 bis 2 : 1.

10. Parfümierter und/oder aromatisierter Artikel, umfassend eine Riechstoff- und/oder Aromastoffmischung nach einem der Ansprüche 7 bis 9.

11. Parfümierter und/oder aromatisierter Artikel nach Anspruch 10, wobei der Artikel ausgewählt ist aus der Gruppe bestehend aus: Parfüm-Extraits, Eau de Parfums, Eau de Toilettes, Rasierwässer, Eau de Colognes, Pre-shave-Produkte, Splash-Colognes, parfümierte Erfrischungstüchern, saure, alkalische und neutrale Reinigungsmitteln, Textilerfrischern, Bügelhilfen, flüssigen Waschmitteln, pulverförmigen Waschmitteln, Wäschevorbehandlungsmitteln, Wäscheweichspülern, Waschseifen, Waschtabletten, Desinfektionsmitteln, Oberflächendesinfektionsmitteln, Luftverbesserern, Aerosolsprays, Wachse und Polituren, Körperpflegemitteln, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkten der dekorativen Kosmetik, Kerzen, Lampenölen, Räucherstäbchen, Insektiziden, Repellentien und Treibstoffen.

12. Verbindung der Formel (I) 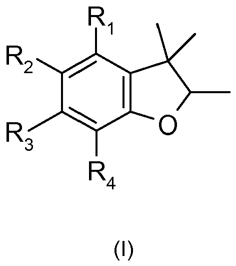 oder Mischung bestehend aus zwei, drei, vier, fünf, sechs oder mehreren verschiedenen Verbindungen der Formel (I),
wobei für die Reste der Verbindung der Formel (I) bzw. die Reste sämtlicher Verbindungen der Formel (I) jeweils unabhängig voneinander gilt:
R₁ bedeutet Wasserstoff, Methyl, tert-Butyl, Acetyl, Formyl oder Cyano, vorzugsweise Wasserstoff oder Methyl,
R₂ bedeutet Wasserstoff, Methyl, tert-Butyl, Formyl, Acetyl oder Cyano, vorzugsweise Formyl oder Acetyl,
R₃ bedeutet Wasserstoff, Methyl oder tert-Butyl, und
R₄ bedeutet Wasserstoff, Methyl, Ethyl, Propyl, Isopropyl, Butyl, sec-Butyl, tert-Butyl, iso-Butyl, Formyl, Acetyl oder Cyano, vorzugsweise Methyl, Ethyl, Isopropyl oder tert-Butyl
mit der Maßgabe, dass die Verbindung(en) der Formel (I) nicht ausgewählt ist bzw. sind aus der Gruppe bestehend aus
2,3-Dihydro-2,3,3-trimethylbenzofuran,
2,3-Dihydro-2,3,3- trimethyl-7-benzofurancarbaldeyd und
2,3-Dihydro-2,3,3,4,7-pentamethyl-benzofuran.

13. Verbindung oder Mischung nach Anspruch 12, wobei für die Reste der Verbindung der Formel (I) bzw. die Reste sämtlicher Verbindungen der Formel (I) jeweils unabhängig voneinander gilt:
R₁ bedeutet Wasserstoff oder Methyl,
R₂ bedeutet Formyl oder Acetyl,
R₃ bedeutet Wasserstoff oder Methyl und
R₄ bedeutet Methyl, Ethyl, Isopropyl oder tert-Butyl.

14. Verbindung oder Mischung nach einem der Ansprüche 12 oder 13, wobei die Verbindung der Formel (I) bzw. eine, mehrere oder sämtliche Verbindungen der Formel (I) ausgewählt ist bzw. jeweils unabhängig voneinander ausgewählt sind aus der Gruppe bestehend aus den folgenden Verbindungen 1 bis 8, vorzugsweise aus den Verbindungen 2, 4 und 5:

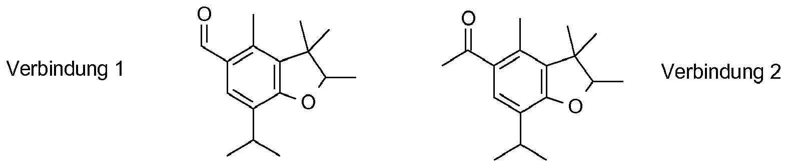

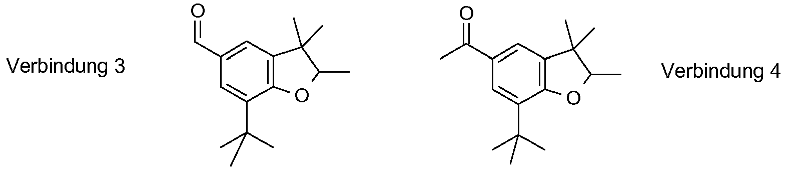

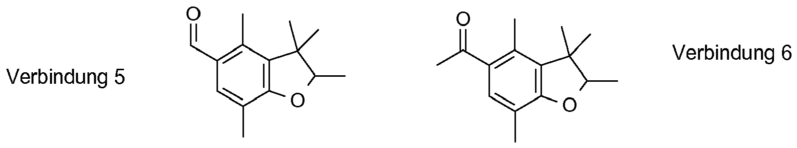

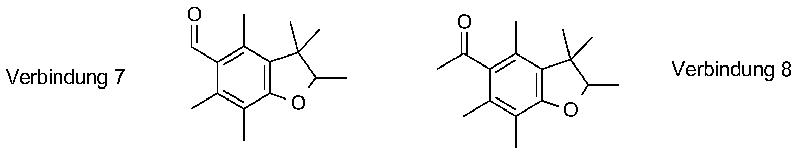

15. Verfahren zum Vermitteln, Modifizieren und/oder Verstärken eines Geruchs und/oder Geschmacks, vorzugsweise eines moschusartigen und/oder holzigen Geruchs und/oder Geschmacks, umfassend folgenden Schritt:
In Kontakt Bringen oder Mischen einer sensorisch wirksamen Menge
- einer Verbindung der Formel (I) 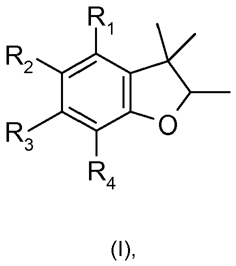 wie in einem der Ansprüche 1 bis 6 oder 12 bis 14 definiert,
- einer Mischung aus zwei, drei, vier, fünf, sechs oder mehreren verschiedenen Verbindungen der Formel (I),
wie in einem der Ansprüche 1 bis 6 oder 12 bis 14 definiert,
oder
- einer Riechstoff- und/oder Aromastoffmischung nach einem der Ansprüche 7 bis 9 mit einem Erzeugnis.

## Claims

1. Use
- of a compound of formula (I) 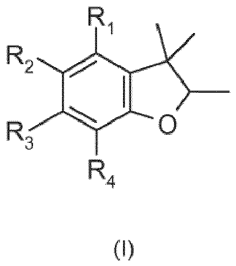 or
- of a mixture of two, three, four, five, six or more different compounds of formula (I)
wherein for the radicals of the compound of formula (I) or respectively for the radicals of all compounds of formula (I) condition is, that R₁, R₂, R₃ and R₄ independently from each other represent hydrogen, methyl, ethyl, propyl, isopropyl, butyl, sec-butyl, tert.-butyl, iso-butyl, acetyl, formyl or cyano,
as a fragrance or aroma compound or as a fragrance or aroma composition.

2. Use of a compound or a mixture according Claim 1 as a fragrance and/or aroma compound with musk-like note or respectively as a fragrance and/or aroma mixture with musk-like note.

3. Use of a compound or a mixture according one of Claims 1 or 2 for the mediation, modification and/or reinforcement of an odour and/or a taste, preferably of a musk-like odour, particularly of a bright musk-like odour, an exalting odour and/or a wood-like, preferably a smooth wood-like odour.

4. Use according to one of the preceding claims, wherein for the compound of formula (I) or respectively for one, more than one or all compounds of formula (I) independently from each other the condition is:
R₁ means hydrogen, methyl, acetyl or formyl, preferably hydrogen or methyl
and/or
R₂ means methyl, formyl or acetyl, preferably formyl or acetyl,
and/or
R₃ means hydrogen or methyl,
and/or
R₄ means methyl, ethyl, propyl, isopropyl, butyl, sec-butyl, tert.-butyl or iso-butyl, preferably methyl, ethyl, isopropyl or tert.-butyl.

5. Use according to one of the preceding claims, wherein for the compound of formula (I) or for one, more than one or all compounds of formula (I) independently from each other condition is:
R₁ means hydrogen, methyl, acetyl or formyl, preferably hydrogen or methyl
and/or
R₂ means methyl, formyl or acetyl, preferably formyl or acetyl,
and/or
R₃ means hydrogen or methyl,
and/or
R₄ means methyl, ethyl, propyl, isopropyl, butyl, sec-butyl, tert.-butyl or iso-butyl, preferably methyl, ethyl, isopropyl or tert.-butyl.

6. Use according to one of the preceding claims, wherein the compound of formula (I) or respectively one, more than one or all compounds of formula (I) is selected or respectively are independently from each other selected from the group consisting of the following compounds 1 to 8:

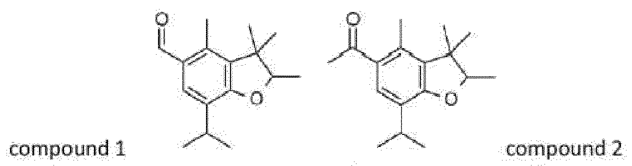

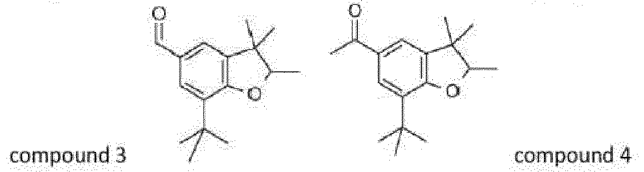

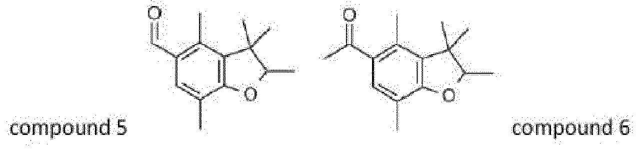

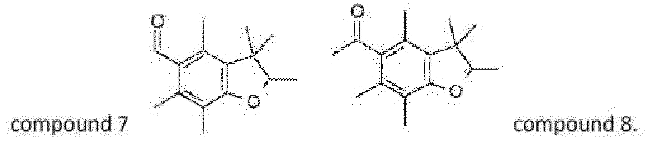

7. Fragrance and/or aroma mixture, comprising or consisting of
(a) a compound of formula (I) or a mixture of two, three, four, five, six or more different compounds of formula (I), as defined in one of the preceding claims, preferably as defined in one of the claims 4 to 6, particularly as defined in Claim 5, and
(b) one, two, three or more additional fragrance and/or aroma compounds, wherein the additional fragrance and/or aroma compound(s) are different from formula (I).

8. Fragrance and/or aroma mixture according Claim7, wherein the fragrance or respectively the aroma compound or respectively one, two, three, or more or all fragrance or respectively aroma compounds according to component (b) provide a wood-like, a musk-like and/or flower-like odour and/or taste.

9. Fragrance and/or aroma mixture according to one of Claims 7 or 8, wherein the total amount of compounds of formula (I) is in the range of 0.00001 to 99.9 % b.w., preferably in the range of 0.001 to 70 % b.w., more preferably in the range of 0.01 to 50 % b.w. - calculated on the total amount of the fragrance or respectively the aroma mixture,
and/or
the ratio by weight of the total amount of component (a) to the total amount of component (b) within the fragrance or respectively aroma mixture ranges from 1 : 100,000 to 10 : 1, preferably 1 : 10,000 to 5 : 1; and more preferably 1 : 1,000 : 2 : 1.

10. Perfumed or aromatized article, comprising a fragrance and/or aroma mixture according to one of Claims 7 to 9.

11. Perfumed of aromatized article according Claim 10, wherein said article is selected from the group consisting of: perfume extracts, eau de parfums, eau de toilettes, aftershaves, eau de Colognes, pre-shave products, splash-Colognes, perfumed refreshing towels, acidic, alkaline or neutral cleaners, textile fresheners, ironing aids, liquid detergents, powder detergents, textile pre-treatment agents, textile softeners, washing soaps, detergent tablets, disinfectants, surface disinfectants, air refresher, aerosol sprays, waxes and polishing agents, personal care products, hand crèmes and lotions, food crèmes and lotions, depilation crèmes and lotions, after-shave crèmes and lotions, skin tanning crèmes and lotions, hair care products, deodorants and antiperspirants, products for decorative cosmetics, candles, lamp oils, incense sticks, insecticides, repellents an fuels.

12. Compound of formula (I) 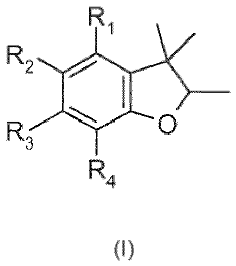 or a mixture consisting of two, three, four, five, six or more different compounds of formula (I)
wherein for the radicals of the compound of formula (I) or respectively for the radicals of all compounds of formula (I) condition is:
R₁ means hydrogen, methyl, tert.-butyl, acetyl, formyl or cyano, preferably hydrogen or methyl,
R₂ means hydrogen, methyl, tert.-butyl, formyl, acetyl or cyano, preferably formyl or acetyl,
R₃ means hydrogen, methyl or tert.-butyl, and
R₄ means methyl, ethyl, propyl, isopropyl, butyl, sec-butyl, tert.-butyl, iso-butyl, formyl, acetyl or cyano, preferably methyl, ethyl, isopropyl or tert.-butyl
on condition, that the compound(s) of formula (I) are not selected from or respectively do not represent the group consisting of
2,3-dihydro-2,3,3-trimethylbenzofuran, 2,3-dihydro-2,3,-trimethyl-7-benzofurancarbaldehyde, and 2,3-dihydro-2,3,3,4,7-pentamethyl-benzofuran.

13. Compound of mixture according Claim 12, wherein for the radicals of component (I) or for the radicals of all compounds of formula (I) independently the condition is:
R₁ means hydrogen or methyl,
R₂ means formyl or acetyl,
R₃ means hydrogen or methyl, and
R₄ means methyl, ethyl, isopropyl or tert.-butyl.

14. Compound or mixture according to one of claims 12 or 13, wherein the compound of formula (I) or respectively one or more or all compounds of formula (I) are selected or respectively independently from each other are selected from the group consisting of the following compounds 1 to 8, preferably compounds 2, 4 and 5:

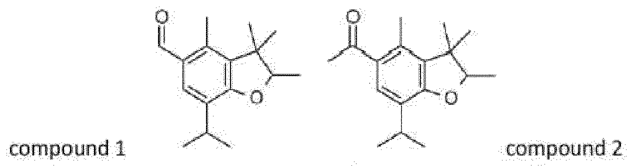

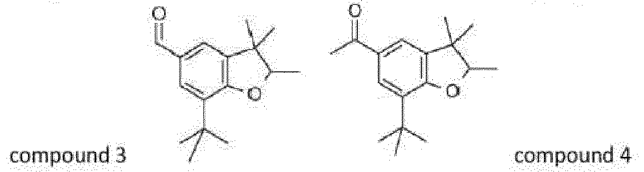

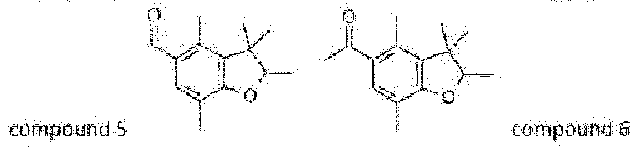

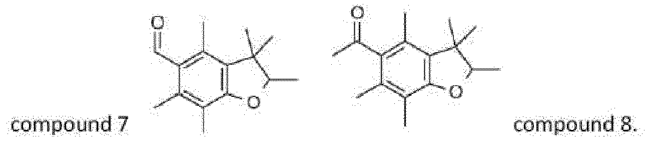

15. Process for mediation, modification and/or reinforcement of an odour and/or a taste, preferably of a musk-like and/or wood-like odour and/or taste, encompassing the following steps:
Bringing in contact or mixing a sensory-active amount
- of a compound of formula (I) 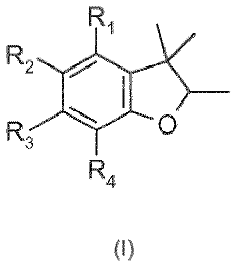 as defined in Claims 1 to 6 or 12 to 14,
- a mixture of two, three, four, five, six or more different compounds of formula (I), as defined in Claims 1 to 6 or 12 to 14, or
- a fragrance and/or aroma mxture according to one of Claim 7 to 9
with an article.

## Revendications

1. Utilisation
- d'un composé de formule (I) 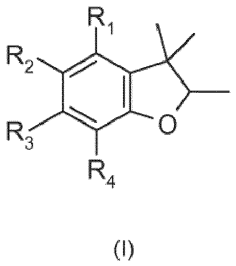 ou
- d'un mélange de deux, trois, quatre, cinq, six composés de formule (I) différents ou plus,
les radicaux du composé de formule (I) ou les radicaux de tous les composés de formule (I) étant à chaque fois tels que R₁, R₂, R₃ et R₄ signifient chacun indépendamment les uns des autres hydrogène, méthyle, éthyle, propyle, isopropyle, butyle, sec-butyle, tert-butyle, iso-butyle, acétyle, formyle ou cyano,
en tant que parfum et/ou arôme ou en tant que mélange de parfums et/ou d'arômes.

2. Utilisation d'un composé ou d'un mélange selon la revendication 1 en tant que parfum et/ou arôme à note musquée ou en tant que mélange de parfums et/ou d'arômes à note musquée.

3. Utilisation d'un composé ou d'un mélange selon l'une quelconque des revendications 1 ou 2 pour la véhiculation, la modification et/ou le renforcement d'une odeur et/ou d'un goût, de préférence d'une odeur musquée, notamment d'une odeur musquée rayonnante, d'une odeur exaltante et/ou d'une odeur boisée, notamment d'une odeur boisée douce.

4. Utilisation selon l'une quelconque des revendications précédentes, dans laquelle, dans le composé de formule (I) ou dans un, plusieurs ou tous les composés de formule (I), à chaque fois indépendamment les uns des autres :
R₁ signifie hydrogène, méthyle, acétyle ou formyle, de préférence hydrogène ou méthyle,
et/ou
R₂ signifie méthyle, formyle ou acétyle, de préférence formyle ou acétyle,
et/ou
R₃ signifie hydrogène ou méthyle,
et/ou
R₄ signifie méthyle, éthyle, propyle, isopropyle, butyle, sec-butyle, tert-butyle ou iso-butyle, de préférence méthyle, éthyle, isopropyle ou tert-butyle.

5. Utilisation selon l'une quelconque des revendications précédentes, dans laquelle, dans le composé de formule (I) ou dans un, plusieurs ou tous les composés de formule (I), à chaque fois indépendamment les uns des autres :
R₁ signifie hydrogène, méthyle, acétyle ou formyle, de préférence hydrogène ou méthyle,
R₂ signifie méthyle, formyle ou acétyle, de préférence formyle ou acétyle,
R₃ signifie hydrogène ou méthyle, et
R₄ signifie méthyle, éthyle, propyle, isopropyle, butyle, sec-butyle, tert-butyle ou iso-butyle, de préférence méthyle, éthyle, isopropyle ou tert-butyle.

6. Utilisation selon l'une quelconque des revendications précédentes, dans laquelle le composé de formule (I) ou un, plusieurs ou tous les composés de formule (I) est choisi ou sont choisis chacun indépendamment les uns des autres dans le groupe constitué par les composés 1 à 8 suivants :

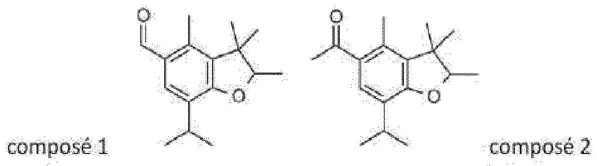

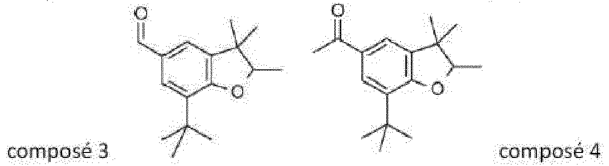

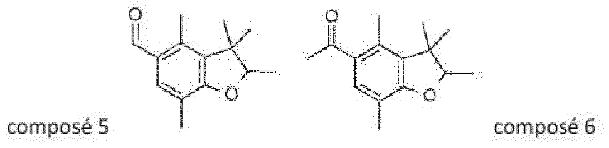

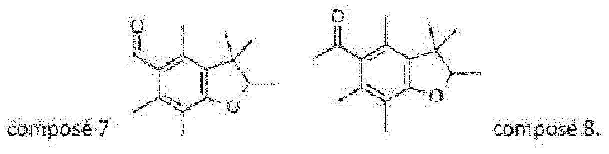

7. Mélange de parfums et/ou d'arômes, comprenant ou constitué par :
(a) un composé de formule (I) ou un mélange de deux, trois, quatre, cinq, six composés de formule (I) différents ou plus, tels que définis à chaque fois dans l'une quelconque des revendications précédentes, de préférence tels que définis à chaque fois dans l'une quelconque des revendications 4 à 6, notamment tels que définis dans la revendication 5,
et
(b) un, deux, trois parfums et/ou arômes supplémentaires ou plus, le ou les parfums ou arômes supplémentaires n'étant pas des composés de formule (I).

8. Mélange de parfums et/ou d'arômes selon la revendication 7, dans lequel le ou un, deux, trois, plus ou tous les parfums ou arômes supplémentaires selon le constituant (b) véhiculent une odeur et/ou un goût boisé, musqué et/ou fleuri.

9. Mélange de parfums et/ou d'arômes selon l'une quelconque des revendications 7 ou 8, dans lequel
la quantité totale des composés de formule (I) se situe dans la plage allant de 0,00001 à 99,9 % en poids, de préférence dans la plage allant de 0,001 à 70 % en poids, de manière particulièrement préférée dans la plage allant de 0,01 à 50 % en poids, par rapport au poids total du mélange de parfums ou d'arômes,
et/ou
le rapport en poids entre la quantité totale de constituant (a) et la quantité totale de constituant (b) dans le mélange de parfums ou d'arômes se situe dans la plage allant de 1:100 000 à 10:1, de préférence dans la plage allant de 1:10 000 à 5:1, notamment dans la plage allant de 1:1000 à 2:1.

10. Article parfumé et/ou aromatisé, comprenant un mélange de parfums et/ou d'arômes selon l'une quelconque des revendications 7 à 9.

11. Article parfumé et/ou aromatisé selon la revendication 10, dans lequel l'article est choisi dans le groupe constitué par : les extraits de parfum, les eaux de parfum, les eaux de toilette, les eaux de rasage, les eaux de Cologne, les produits de prérasage, les eaux de Cologne en flacon, les pochettes rafraichissantes parfumées, les produits de nettoyage acides, alcalins et neutres, les rafraichissants pour textiles, les auxiliaires de repassage, les produits de nettoyage liquides, les produits de nettoyage en poudre, les produits de prélavage, les adoucissants de lavage, les savons de lavage, les tablettes de lavage, les désinfectants, les désinfectants de surfaces, les désodorisants, les sprays aérosols, les cires et les produits de polissage, les produits de soin pour le corps, les crèmes et lotions pour les mains, les crèmes et lotions pour les pieds, les crèmes et lotions de dépilation, les crèmes et lotions après-rasage, les crèmes et lotions de brunissement, les produits de soin pour les cheveux, les déodorants et les antiperspirants, les produits de cosmétique décorative, les bougies, les huiles pour lampes, les bâtons d'encens, les insecticides, les répulsifs et les carburants.

12. Composé de formule (I) 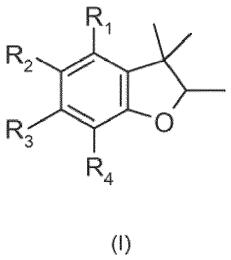 ou mélange constitué par deux, trois, quatre, cinq, six composés de formule (I) différents ou plus,
les radicaux du composé de formule (I) ou les radicaux de tous les composés de formule (I) étant à chaque fois indépendamment les uns des autres tels que :
R₁ signifie hydrogène, méthyle, tert-butyle, acétyle, formyle ou cyano, de préférence hydrogène ou méthyle,
R₂ signifie hydrogène, méthyle, tert-butyle, formyle, acétyle ou cyano, de préférence formyle ou acétyle,
R₃ signifie hydrogène, méthyle ou tert-butyle, et
R₄ signifie hydrogène, méthyle, éthyle, propyle, isopropyle, butyle, sec-butyle, tert-butyle, iso-butyle, formyle, acétyle ou cyano, de préférence méthyle, éthyle, isopropyle ou tert-butyle,
à condition que le ou les composés de formule (I) ne soient pas choisis dans le groupe constitué par :
le 2,3-dihydro-2,3,3-triméthylbenzofurane,
le 2,3-dihydro-2,3,3-triméthyl-7-benzofurane-carbaldéhyde et
le 2,3-dihydro-2,3,3,4,7-pentaméthyl-benzofurane.

13. Composé ou mélange selon la revendication 12, dans lequel les radicaux du composé de formule (I) ou les radicaux de tous les composés de formule (I) sont à chaque fois indépendamment les uns des autres tels que :
R₁ signifie hydrogène ou méthyle,
R₂ signifie formyle ou acétyle,
R₃ signifie hydrogène ou méthyle, et
R₄ signifie méthyle, éthyle, isopropyle ou tert-butyle.

14. Composé ou mélange selon l'une quelconque des revendications 12 ou 13, dans lequel le composé de formule (I) ou un, plusieurs ou tous les composés de formule (I) est choisi ou sont choisis chacun indépendamment les uns des autres dans le groupe constitué par les composés 1 à 8 suivants, de préférence les composés 2, 4 et 5 :

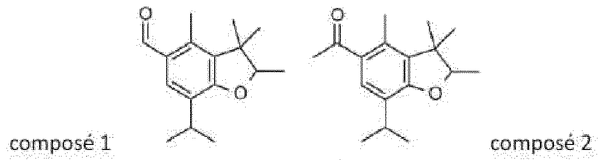

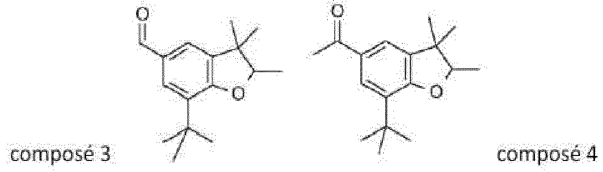

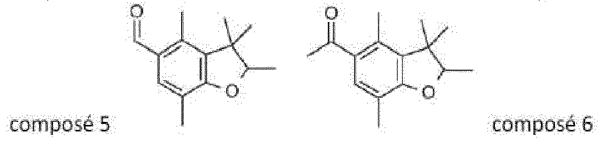

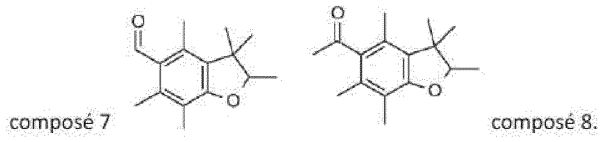

15. Procédé de véhiculation, de modification et/ou de renforcement d'une odeur et/ou d'un goût, de préférence d'une odeur et/ou d'un goût musqué et/ou boisé, comprenant l'étape suivante :
la mise en contact ou le mélange d'une quantité sensoriellement efficace
- d'un composé de formule (I) 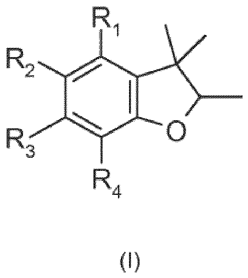 tel que défini dans l'une quelconque des revendications 1 à 6 ou 12 à 14,
- d'un mélange de deux, trois, quatre, cinq, six composés de formule (I) différents ou plus,
tel que défini dans l'une quelconque des revendications 1 à 6 ou 12 à 14,
ou
- d'un mélange de parfums et/ou d'arômes selon l'une quelconque des revendications 7 à 9,
avec un article.
